# EUROPEAN PATENT APPLICATION

(11) **EP 4 406 937 A1**
(43) Date of publication of application: **31.07.2024**
(21) Application number: 22871956.3
(22) Date of filing: 20.09.2022
(51) Int. Cl.: C07C 323/52, C07C 381/10, C07D 305/08, C07D 285/10, C07D 285/16, C07D 249/08, C07D 257/04, C07D 333/34, A61K 31/192, A61K 31/337, A61K 31/381, A61K 31/265, A61K 31/222, A61K 31/4196, A61K 31/41, A61K 31/549, A61K 31/433, A61P 43/00

(54) **SMALL-MOLECULE COMPOUND HAVING NAPHTHOL ETHER STRUCTURE, AND USE THEREOF**

(30) Priority: 22.09.2021 CN 202111106864
(71) Applicant: Hangzhou Phecdamed Co., Ltd., Hangzhou, Zhejiang 311100 (CN)
(72) Inventor: XIA, Hongguang, Hangzhou, Zhejiang 311100 (CN); FAN, Mengyang, Hangzhou, Zhejiang 311100 (CN); CEN, Xufeng, Hangzhou, Zhejiang 311100 (CN); XU, Xiaoyan, Hangzhou, Zhejiang 311100 (CN); WU, Ronghai, Hangzhou, Zhejiang 311100 (CN); LIU, Dong, Hangzhou, Zhejiang 311100 (CN); TIAN, Zhen, Hangzhou, Zhejiang 311100 (CN)
(74) Representative: Viering, Jentschura & Partner mbB Patent- und Rechtsanwälte
(86) International application number: PCT/CN2022/119826
(87) International publication number: WO 2023/045910

(57) **Abstract**

The present application discloses a small-molecule compound having a naphthalenethiol ether structure, and a use thereof. Disclosed in the present application are a compound having a structure represented by general formula (I), a pharmaceutically acceptable salt, a stereoisomer, a solvate or a prodrug thereof, a pharmaceutical composition containing the same, and a use thereof in the preparation of mitophagy inducer drugs. In the present application, the structure of the compound having the structure represented by general formula (I) is shown below. The compound and the pharmaceutically acceptable salt, stereoisomer, solvate or prodrug thereof, or the pharmaceutical composition provided in the present application can enhance the ability to induce mitophagy.

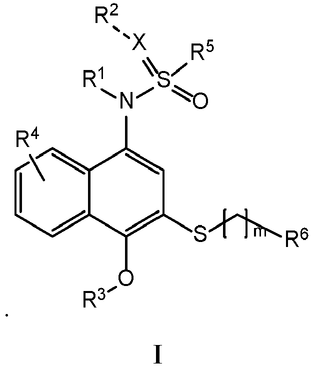

## Description

### Cross-references to related applications

This patent application claims priority to the Chinese patent application No. 2021111068648 Filed on September 22, 2021, and entitled "small-molecule compound having naphthalenethiol ether structure, and use thereof", which is incorporated herein by reference in its entirety.

### Technical field

The invention relates to the field of chemical drugs, and in particular to a small-molecule compound having naphthalenethiol ether structure and use thereof.

### Background

As one type of cellular target-specific autophagy, the main purpose of mitophagy is to identify and remove dysfunctional mitochondria. Since mitochondria play a central role in energy supply through oxidative phosphorylation and have important functions including energy metabolism, amino acid production, lipid synthesis, and ion homeostasis, which are very important for maintaining the function of cell types that depend on aerobic metabolism, such as neuronal cells, muscle cells, and hepatocytes. Homeostatic regulation of mitochondrial production and autophagy is an important step in maintaining cellular function. Mitophagy dysfunction will lead to the accumulation of damaged mitochondria, a decrease in the ability to synthesize ATP+, and the production of a large amount of peroxide, which will lead to changes in cellular intermediate metabolites and trigger a series of pathologic consequences. If aging or dysfunctional mitochondria are cleared by enhancing mitophagy, mitophagy will play a protective role in cells.

In the prior art, the Chinese patent application No. 2 01910386493.X describes a UMI-77 as a mitophagy inducer. However, the inventors have found in their research that UMI-77, as a mitophagy inducer, has problems such as unstable mitophagy-inducing effect, insufficient ability to induce mitophagy, and poor metabolic stability. Therefore, the development of an efficient and stable mitophagy inducer is crucial to inhibit or alleviate various acute and chronic diseases caused by mitophagy dysfunction .

### Summary of the invention

In order to solve the above problems, embodiments of the present invention provide a class of small molecule compounds with a naphthiophenol ether structure, which have a stronger ability to induce mitophagy as a mitophagy inducer.

In a first aspect of the present invention, it provides a compound having a structure represented by a general formula (I) and a pharmaceutically acceptable salt, stereoisomer, solvate or prodrug thereof. 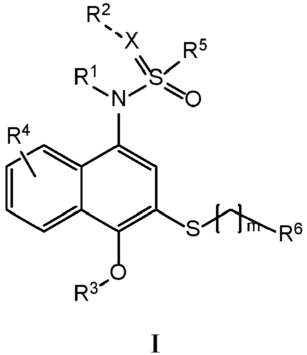
wherein X is oxygen or nitrogen;
R³ is hydrogen, 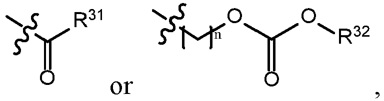 wherein R³¹ and R³² are each independently C_{1 ~ 6} alkyl or C _{1 ∼ 6} cycloalkyl, n is 1 ~ 4 .

In each general formula and context of this specification, unless explicitly stated, R⁴ may not exist (that is, the number thereof is 0), or one or more R⁴ may exist at the same time if the valence bond rules allow, so that the number of R⁴ may be, for example, 0~5, preferably 0~4, more preferably 0~2 or 0~1. When the number of R⁴ is not 0, each is independently selected from halogen, nitro, nitrile , -N⁺(R⁴⁻¹)₃, C_{1∼6} haloalkyl, -C(O)OR⁴⁻¹ , -C(O)R⁴⁻¹ , -C(O)N(R⁴⁻¹ R^{4-1a}) , -S(O)₂R⁴⁻¹ , - S(O)R⁴⁻¹, -N=C(R⁴⁻¹R^{4-1a}) , wherein R⁴⁻¹ and R^{4-1a} are each independently hydrogen, C_{1~6} alkyl, C₂₋₆ alkenyl, C₂₋₆ alkynyl , C₁₋₆ alkyl with at least one hydrogen substituted by halogen , C ₂₋₆ alkenyl with at least one hydrogen substituted by halogen , or C ₂₋₆ alkynyl with at least one hydrogen substituted by halogen .
R⁵ is phenyl, naphthyl, 5- or 6-membered monocyclic heteroaryl, 8 to 10- membered fused bicyclic heteroaryl, phenyl with at least one hydrogen atom substituted by R⁵⁻¹ , naphthyl with at least one hydrogen atom substituted by R⁵⁻¹, 5- or 6-membered monocyclic heteroaryl with at least one hydrogen atom substituted by R⁵⁻¹ , 8- to 10-membered fused bicyclic heteroaryl with at least one hydrogen atom substituted by R⁵⁻¹ , wherein R⁵⁻¹ is hydrogen, halogen, nitro, nitrile , hydroxyl, C_{1~6} alkyl, C_{3∼6} cycloalkyl, C_{1~6} alkoxy , -N(R^{5 -1a} R^{5-1b}), phenyl, C_{1~6} haloalkyl, C_{1~6} haloalkoxy, - C(O)OR⁵⁻¹¹, -C(O)R⁵⁻¹¹ , -C(O)N(R^{5-1a}R^{5-1b}), -S(O)₂R⁵⁻¹¹ , -S(O)R⁵⁻¹¹ , - OC(O)R⁵⁻¹¹, -OC(O)OR⁵⁻¹¹ or 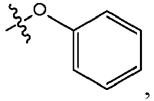 wherein R⁵⁻¹¹ , R^{5-1a} and R^{5-1b} are each independently hydrogen, C_{1~6} alkyl, three to six-membered cycloalkyl, C_{2 - 6} alkenyl, C₂₋₆ alkynyl, C_{1~6} alkyl with at least one hydrogen substituted by halogen , C₂₋₆ alkenyl with at least one hydrogen substituted by halogen, C_{3∼6} cycloalkyl with at least one hydrogen substituted by halogen , C₂₋₆ alkynyl with at least one hydrogen substituted by halogen;
R⁶ is -C(O)OR⁶⁻¹, 5 or 6-membered monocyclic heteroaryl , wherein R⁶⁻¹ is hydrogen, C_{1~6} alkyl, three to six-membered cycloalkyl, C₂₋₆ alkenyl, C₂₋₆ alkynyl , C_{1~6} alkyl with at least one hydrogen substituted by halogen, C₂₋₆ alkenyl with at least one hydrogen substituted by halogen, C_{3∼6} cycloalkyl with at least one hydrogen substituted by halogen, C₂₋₆ alkynyl with at least one hydrogen substituted by halogen;
m is 1~6 ; and
when X is oxygen, the dotted line does not exist and R² does not exist, R¹ is C_{1~6} alkyl, three- to six-membered cycloalkyl, three- to six-membered epoxyalkyl, phenyl, C_{1~6} alkyl with at least one hydrogen substituted by R¹⁻¹ , and phenyl with at least one hydrogen substituted by R¹⁻¹ , wherein R¹⁻¹ is hydroxyl, halogen, amino or C_{1~6} alkoxy;
when X is nitrogen, the dotted line is a single bond,
R¹ is hydrogen, C_{1~6} alkyl, three to six-membered cycloalkyl, three to six-membered epoxyalkyl, phenyl, C_{1∼6} alkyl with at least one hydrogen substituted by R¹⁻¹ , phenyl with at least one hydrogen substituted by R¹⁻² , where R¹⁻² is hydroxyl, halogen, amino or C_{1~6} alkoxy,
R² is a C_{1~6} alkyl, a three- to six-membered cycloalkyl, a three- to six-membered epoxyalkyl, or a C_{1~6} alkyl in which at least one hydrogen is substituted by R²⁻¹ , wherein R²⁻¹ is hydroxyl, halogen, amino or C_{1∼6} alkoxy;
when X is nitrogen and R¹ and R² are independently C₁₋₆ alkyl, R¹ and R² can be bonded to form a 4~8-membered ring.

In some preferred embodiments, in the compound having the structure represented by the general formula (I) ,
X is oxygen or nitrogen;
R³ is hydrogen, 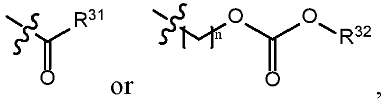 wherein R³¹ and R³² are each independently C_{1~4} alkyl or C_{1~4} cycloalkyl, n is 1 or 2;
when the number of R⁴ is not 0, each is independently selected from halogen, nitro, nitrile , -N⁺(R⁴⁻¹)₃, C_{1~4} haloalkyl, -C(O)OR⁴⁻¹ , -C(O)R⁴⁻¹ , -C(O)N(R⁴⁻¹ R^{4-1a}) , -S(O)₂R⁴⁻¹ , -S(O)R⁴⁻¹ , -N=C(R⁴⁻¹ R^{4-1a}) , wherein R⁴⁻¹ and R^{4-1a} are each independently hydrogen, C₁₋₄alkyl, C₂₋₄ alkenyl, C₂₋₄ alkynyl ;
R⁵ is phenyl, naphthyl, 5- or 6-membered monocyclic heteroaryl, phenyl with at least one hydrogen atom substituted by R⁵⁻¹ , naphthyl with at least one hydrogen atom substituted with R⁵⁻¹ , 5- or 6-membered monocyclic heteroaryl with at least one hydrogen atom substituted by R⁵⁻¹ , wherein R⁵⁻¹ is hydrogen, halogen, nitro, nitrile , hydroxyl, C_{1∼4} alkyl, or three to six-membered cycloalkyl, C_{1~4} alkoxy, -N(R^{5-1a} R^{5-1b}), phenyl, C_{1~4} haloalkyl, C_{1∼4} haloalkoxy, -C(O)OR⁵⁻¹¹ , -C(O)R⁵⁻¹¹ , -C(O)N(R^{5-1a} R^{5-1b}) , -S(O)₂R⁵⁻¹¹ , -S(O)R⁵⁻¹¹ , - OC(O)R⁵⁻¹¹ , -OC(O)OR⁵⁻¹¹ or 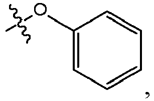 wherein R⁵⁻¹¹ , R^{5-1a} and R^{5-1b} are each independently hydrogen, C_{1~4} alkyl, three to six -membered cycloalkyl, C₂₋₄ alkenyl, C₂₋₄ alkynyl, C_{1~4} alkyl with at least one hydrogen substituted by halogen;
R⁶ is -C(O)OR⁶⁻¹ , 5 or 6-membered monocyclic heteroaryl , wherein R⁶⁻¹ is hydrogen, C_{1~4} alkyl, three to six-membered cycloalkyl, C_{1~4} alkyl with at least one hydrogen substituted by halogen;
m is 1~4 ; and
when X is oxygen, the dotted line does not exist, R² does not exist, and R¹ is C_{1~4} alkyl, three- to six-membered cycloalkyl, three- to six-membered epoxyalkyl, phenyl, C_{1~4} alkyl with at least one hydrogen substituted by R¹⁻¹, and phenyl with at least one hydrogen substituted by R¹⁻¹, wherein R¹⁻¹ is hydroxyl, halogen, amino, or C_{1~4} alkoxy;
when X is nitrogen, the dotted line is a single bond,
R¹ is hydrogen, C_{1~4} alkyl, three to six-membered cycloalkyl, three to six-membered epoxyalkyl, phenyl, C_{1∼4} alkyl with at least one hydrogen substituted by R¹⁻¹ , phenyl with at least one hydrogen substituted by R¹⁻² , wherein R¹⁻² is hydroxyl, halogen, amino or C_{1~4} alkoxy,
R² is C_{1~4} alkyl, three- to six-membered cycloalkyl, three- to six-membered epoxyalkyl, or C_{1~4} alkyl with at least one hydrogen substituted by R²⁻¹ , wherein R²⁻¹ is hydroxyl, halogen, amino or C_{1~4} alkoxy;
when X is nitrogen and R¹ and R² are independently C_{1~4} alkyl, R¹ and R² can be bonded to form a 4~8- membered ring.

In some preferred embodiments, in the compound having the structure represented by the general formula (I) ,
X is oxygen or nitrogen;
R³ is hydrogen, 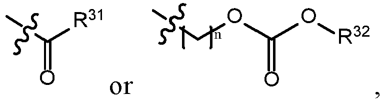 wherein R³¹ and R³² are independently methyl, ethyl, n-propyl or isopropyl, n is 1;
when the number of R⁴ is not 0 , each is independently selected from fluorine, chlorine, bromine, iodine , nitro, nitrile, fluoromethyl, fluoroethyl, fluoro -n- propyl, fluoroisopropyl , chloromethyl, chloroethyl, chloro-n-propyl, chloroisopropyl, bromomethyl, bromoethyl, bromo-n-propyl, bromoisopropyl, iodomethyl, iodoethyl, iodo-n-propyl or iodoisopropyl;
R⁵ is phenyl, naphthyl, 5-membered monocyclic heteroaryl, and phenyl with at least one hydrogen atom substituted by R⁵⁻¹ , wherein R⁵⁻¹ is fluorine, chlorine, bromine, iodine , methoxy, ethoxy, n-propoxy , isopropoxy, n-butoxy , tert-butoxy , sec-butoxy or isobutoxy , phenyl, fluoromethyl, fluoroethyl, fluoro-n-propyl , fluoroisopropyl , chloromethyl, chloroethyl, chloro-n-propyl, chloroisopropyl , bromomethyl , bromoethyl, bromo-n-propyl , bromoisopropyl , iodomethyl, iodoethyl, iodo-n-propyl , iodoisopropyl , fluoromethoxy, fluoroethoxy , fluoro-n-propoxy , fluoroisopropoxy, chloromethoxy, chloroethoxy, chloro-n-propoxy, chloroisopropoxy, bromomethoxy, bromoethoxy, bromo-n-propoxy, bromoisopropoxy, iodooxymethyl, iodoethoxy, iodo-n-propoxy, iodoisopropoxy or 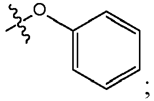
R⁶ is -C(O)OR⁶⁻¹ , five-membered nitrogen-containing monocyclic heteroaryl, preferably pyridyl, imidazole, triazolyl or tetrazolyl , and more preferably triazolyl or tetrazolyl , even more preferably 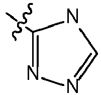 or 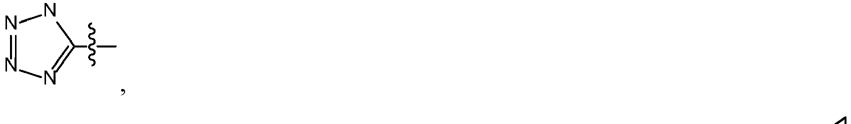
wherein R⁶⁻¹ is hydrogen, methyl, ethyl, n-propyl, isopropyl, 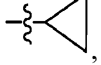 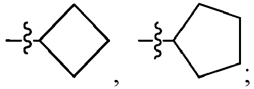
m is 1; and
when X is oxygen, the dotted line does not exist, R² does not exist, and R¹ is methyl, ethyl, n-propyl, or isopropyl, 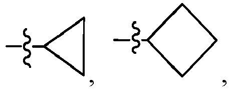 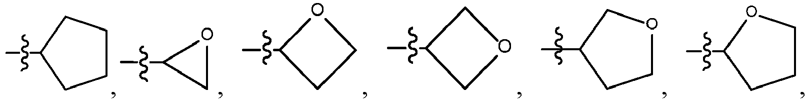 phenyl, methyl with at least one hydrogen substituted by hydroxyl, ethyl with at least one hydrogen substituted by hydroxyl, n-propyl with at least one hydrogen substituted by hydroxyl, isopropyl with at least one hydrogen substituted by hydroxyl;
when X is nitrogen, the dotted line is a single bond,
R¹ is hydrogen, methyl, ethyl, n-propyl , isopropyl, 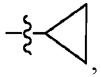 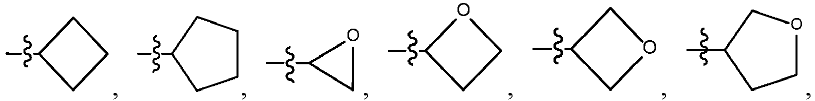 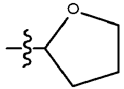 or phenyl,
R² is methyl, ethyl, n-propyl , isopropyl, 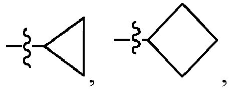 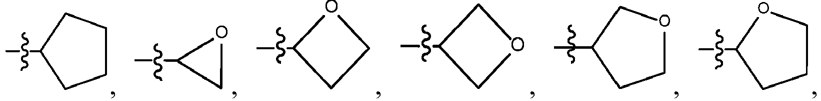 methyl with at least one hydrogen substituted by hydroxyl, ethyl with at least one hydrogen substituted by hydroxyl, n-propyl with at least one hydrogen substituted by hydroxyl or isopropyl with at least one hydrogen substituted by hydroxyl;
when X is nitrogen and R¹ and R² are independently any one of methyl, ethyl, n-propyl or isopropyl, R¹ and R² can be bonded to form a 4-to 8-membered ring.

In some preferred embodiments, the C_{1~6} alkyl is C_{1~4} alkyl, and the C_{1~4} alkyl is preferably methyl, ethyl, n-propyl , isopropyl, n-butyl , isobutyl or tert-butyl , for example: methyl or ethyl.

In some preferred embodiments, the C_{3~6} cycloalkyl is preferably C_{3∼5} cycloalkyl, and the C_{3∼5} cycloalkyl is preferably 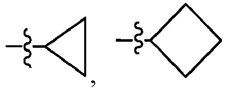 or 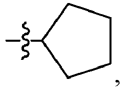 for example:

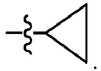

In some preferred embodiments, the three- to six-membered epoxyalkyl is preferably 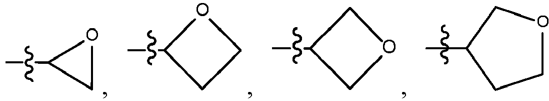 or 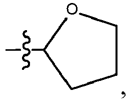 for example:

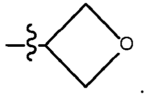

In some preferred embodiments, the phenyl substituted by C_{1~6} alkyl is preferably a phenyl substituted by C_{1~4} alkyl, more preferably a phenyl substituted by any one of methyl, ethyl, n-propyl, isopropyl ,n-butyl, isobutyl and tert-butyl.

In some preferred embodiments, the C_{1~6} alkoxy is preferably a C_{1~4} alkoxy , more preferably methoxy, ethoxy, n-propoxy, isopropoxy, n-butoxy, tert-butoxy, sec-butoxy or isobutoxy.

In some preferred embodiments, -N(R³⁻²R^{3-2a}), -N(R^{3-3a} R^{3-3b} ), -N(R^{4-1a} R^{4-1b}), -N(R⁵⁻¹R^{5-1a}) is preferably 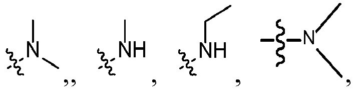 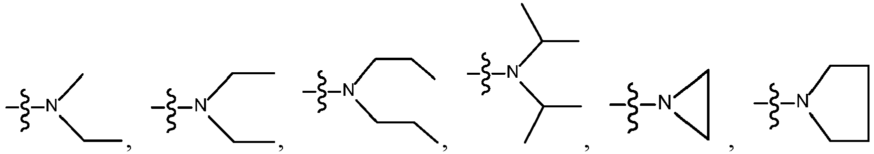 or

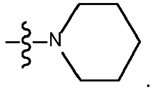

In some preferred embodiments, the 5- or 6-membered monocyclic heteroaryl is preferably 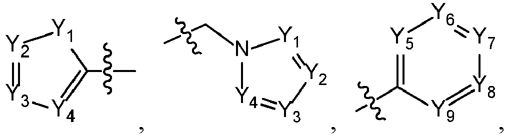 wherein Y₁, Y₂, Y₃, Y₄, Y₅, Y₆, Y₇, Y₈, Y₉ are each independently selected from C, N, O or S, and Y₁, Y₂, Y₃, and Y₄ are not all C, and Y₅, Y₆, Y₇, Y₈, and Y₉ are not all C; the 5- or 6-membered monocyclic heteroaryl is more preferably pyrrolyl, furyl, thienyl, pyrazolyl, imidazolyl, oxazolyl, isoxazolyl, thiazolyl, pyridyl, pyranyl, thiapyran, pyridazinyl, pyrimidinyl, pyrazinyl, piperazinyl, triazolyl, tetrazolyl; the 5- or 6 - membered monocyclic heteroaryl is even more preferably 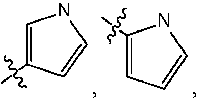 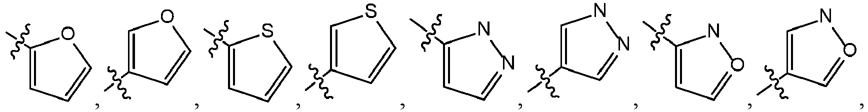 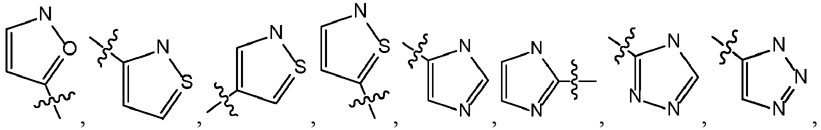 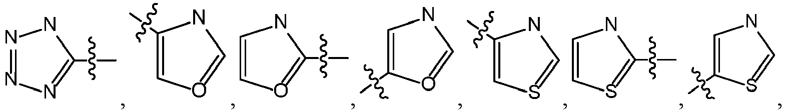 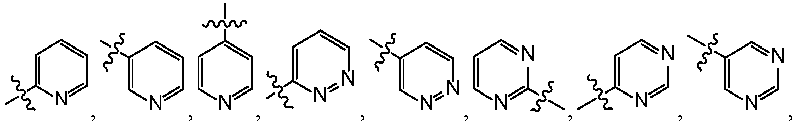 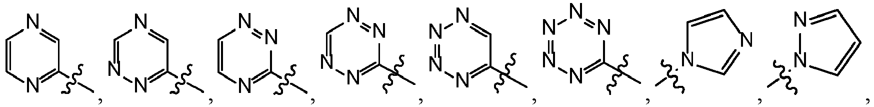 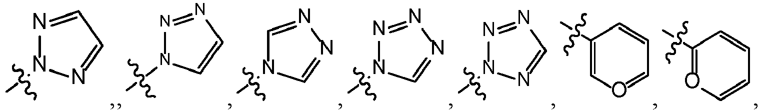 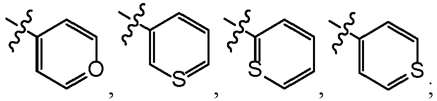 even more preferably, the 5- or 6-membered monocyclic heteroaryl is a 5- or 6-membered nitrogen-containing monocyclic heteroaryl, for example: 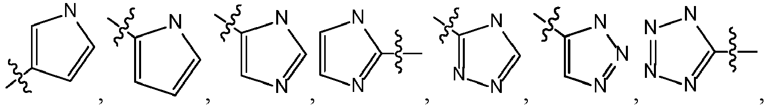 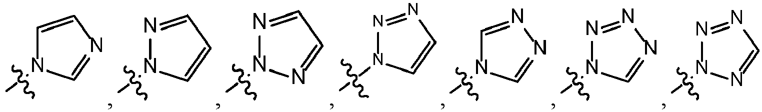 or 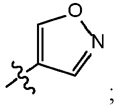 most preferably, the 5- or 6-membered monocyclic heteroaryl is a 5-membered nitrogen-containing monocyclic heteroaryl, such as:

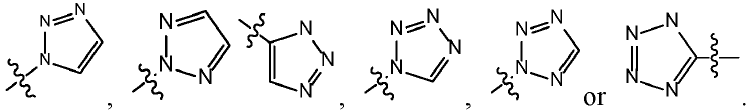

In some preferred embodiments, the 8- to 10-membered fused bicyclic heteroaryl is 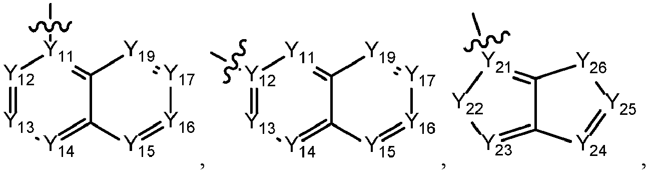 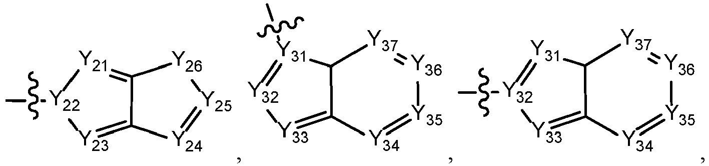 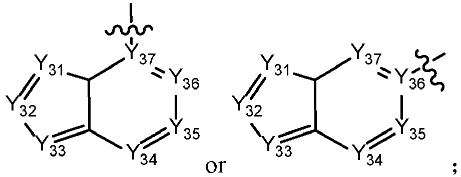 wherein Y₁₁ , Y₁₂, Y₁₃, Y₁₄, Y₁₅, Y₁₆, Y₁₇, Y₁₈ and Y₁₉ are each independently selected from C, N, O or S, and Y₁₁ , Y₁₂, Y₁₃, Y₁₄, Y₁₅, Y₁₆, Y₁₇, Y₁₈ and Y₁₉ are not all C ; Y₂₁, Y₂₂, Y₂₃, Y₂₄, Y₂₅ and Y₂₆ are each independently selected from C, N, O or S, and Y₂₁, Y₂₂, Y₂₃, Y₂₄, Y₂₅ and Y ₂₆ are not all C; Y₃₁, Y₃₂, Y₃₃, Y₃₄, Y₃₅, Y₃₆ and Y₃₇ are each independently selected from C, N, O or S, and Y₃₁, Y₃₂, Y₃₃, Y₃₄, Y₃₅, Y₃₆ and Y₃₇ are not all C; the 8 to 10-membered fused bicyclic heteroaryl is more preferably indolyl, benzindolyl, carbazolyl , quinolyl, pteridyl , purinyl; the 8 to 10-membered fused bicyclic heteroaryl is most preferably

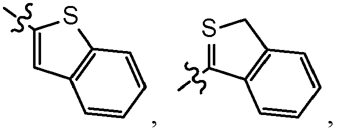

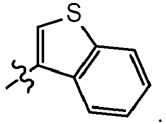

In some preferred embodiments, the halogen is preferably fluorine, chlorine, bromine or iodine.

In some preferred solutions, the C_{1~6} haloalkyl is preferably a C_{1~3} haloalkyl; more preferably, fluoromethyl , fluoroethyl , fluoro-n-propyl , fluoroisopropyl , chloromethyl, chloroethyl, chloro-n-propyl, chloroisopropyl, bromomethyl, bromoethyl, bromo-n-propyl, bromoisopropyl, iodomethyl, iodoethyl, iodo-n-propyl, iodoisopropyl; most preferably, trifluoromethyl.

In some preferred embodiments, the C₁₋₆ haloalkoxy is preferably a C₁₋₃ haloalkoxy; more preferably, fluoromethoxy, fluoroethoxy , fluoro-n-propoxy, fluoroisopropoxy, chloromethoxy, chloroethoxy, chloro-n-propoxy, chloroisopropoxy, bromomethoxy, bromoethoxy, bromo-n-propoxy, bromoisopropoxy, iodooxymethyl, iodoethoxy , iodo-n-propoxy , iodoisopropoxy ; most preferrably, trifluoromethoxy.

In some preferred embodiments, the C₂₋₆ alkenyl is preferably C₂₋₄ alkenyl, more preferably -CH=CH₂, -CH=CH-CH₃, -CH₂-CH=CH₂, -CH=CH-CH₂-CH₃, -CH=CH-CH=CH₂.

In some preferred embodiments, the C₂₋₆ alkynyl is preferably C₂₋₆ alkynyl, more preferably -C=CH, -CH₂-C≡CH, -CH₂-CH₂-C≡CH , -CH₂-C≡C-CH₃ .

In some more preferred embodiments, R¹ is selected from hydrogen, C_{1~6} alkyl (preferably methyl), three to six-membered cycloalkyl (preferably cyclopropyl), three to six-membered epoxyalkyl ( preferably oxetane), phenyl, C₁₋₆ alkyl with at least one hydrogen substituted by R¹⁻¹ (as defined in the context of this specification, preferably hydroxyl) .

In some more preferred embodiments, when the number of R⁴ is not 0, each is independently selected from nitrile, amino, nitro, halogen , and C₁₋₆ haloalkyl.

In some more preferred embodiments, R⁵ is selected from phenyl, naphthyl, 5- or 6-membered monocyclic heteroaryl (such as thienyl, pyrazolyl, isoxazolyl, pyridyl) , 8 to 10-membered fused bicyclic heteroaryl (such as thianaphthene), preferably phenyl or naphthyl ; which is optionally substituted by a group selected from halogen, phenyl, nitrile , hydroxyl, C_{1~6} alkyl, C_{1~6} haloalkyl ,

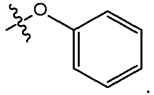

In some more preferred embodiments, m is 1.

In some more preferred embodiments, R⁶ is selected from -C(O)OR⁶⁻¹ ( R⁶⁻¹ is as defined in the context of this specification, preferably hydrogen) and 5- or 6-membered monocyclic heteroaryl (preferably triazolyl and tetrazolyl) .

In some more preferred embodiments, X is nitrogen, and R² is selected from C_{1~6} alkyl (preferably methyl), three- to six-membered cycloalkyl (preferably cyclopropyl), three- to six-membered epoxyalkyl (preferably oxetane), phenyl, C_{1∼6} alkyl with at least one hydrogen substituted by R¹⁻¹ (preferably hydroxyl, as defined in the context of the present specification). In particular, in some of these preferred embodiments, both R¹ and R² are selected from C_{1~6} alkyl, and R¹ and R² are bonded to form a 4- to 8-membered (preferably 5- or 6-membered) ring.

In some preferred embodiments, R³ is hydrogen, 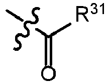 or 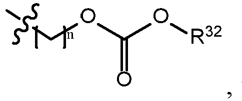 wherein R³¹ and R³² are each independently methyl, n-propyl or isopropyl, and n is 1.

In some preferred embodiments, the number of R⁴ is 0~2 (especially 0~1), and when the number of R⁴ is not 0, each is independently selected from fluorine, chlorine, bromine, iodine , nitro, and nitrile .

In some preferred embodiments, R⁵ is phenyl, naphthyl, thienyl or phenyl with at least one hydrogen atom substituted by R⁵⁻¹ , wherein R⁵⁻¹ is fluorine, chlorine, bromine, iodine , phenyl, fluoromethyl or

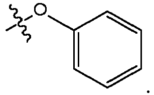

In some preferred embodiments, R⁶ is -C(O)OH , triazolyl or tetrazolyl .

In some preferred embodiments, the compound having the structure represented by the general formula (I) is selected from any one of the following compounds:

| | |
|---|---|
| 1 | 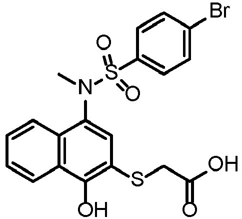 |
| 2 | 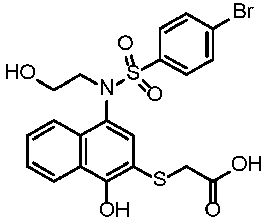 |
| 3 | 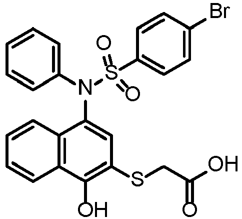 |
| 4 | 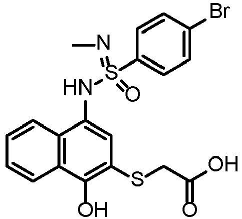 |
| 5 | 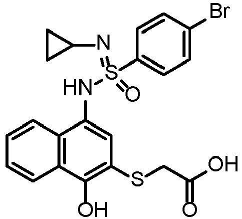 |
| 6 | 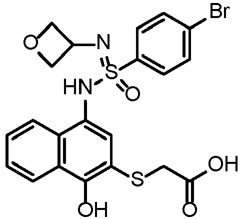 |
| 7 | 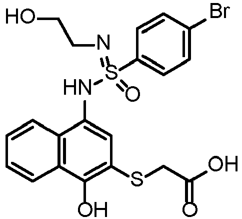 |
| 8 | 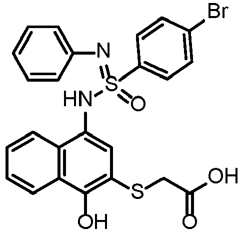 |
| 9 | 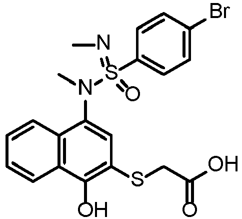 |
| 10 | 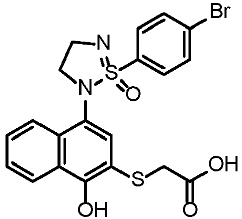 |
| 11 | 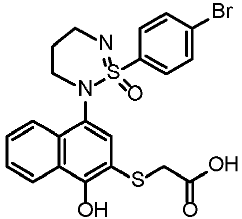 |
| 12 | 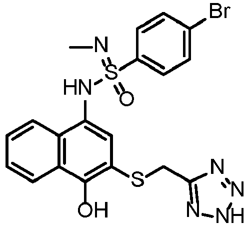 |
| 13 | 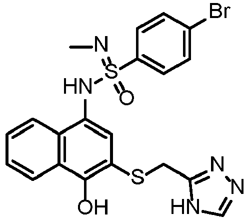 |
| 14 | 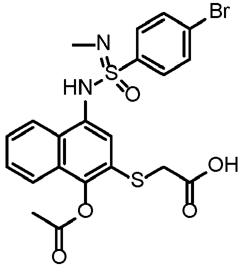 |
| 15 | 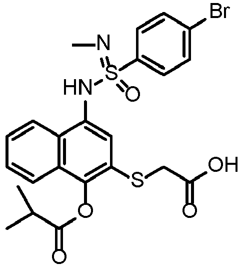 |
| 16 | 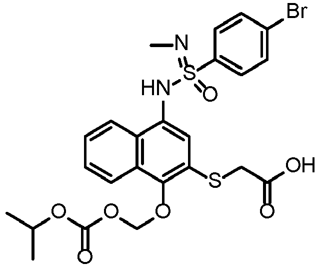 |
| 17 | 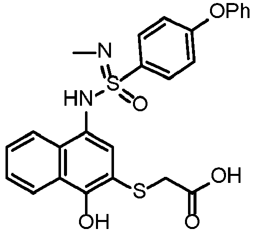 |
| 18 | 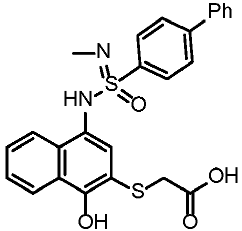 |
| 19 | 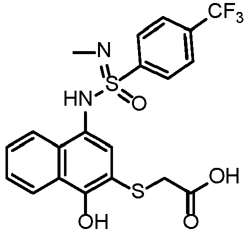 |
| 20 | 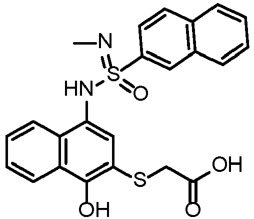 |
| 21 | 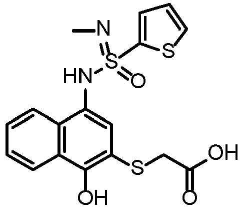 |
| 22 | 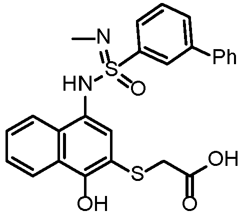 |
| 23 | 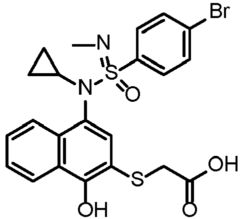 |
| 24 | 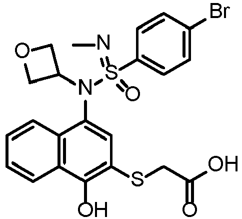 |
| 25 | 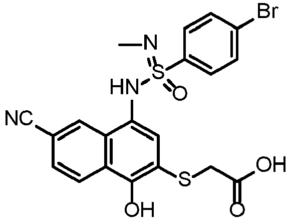 |
| 26 | 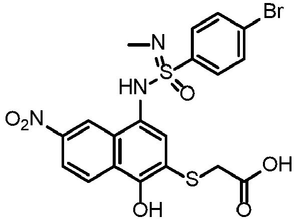 |

Furthermore, the inventors have also found that UMI -77 cannot selectively induce autophagy in damaged mitochondria, based on the fact that it is beneficial for the compound to improve the ability to selectively induce autophagy in damaged mitochondria, in some preferred solutions, the compound has a structurerepresented by a formula (II); 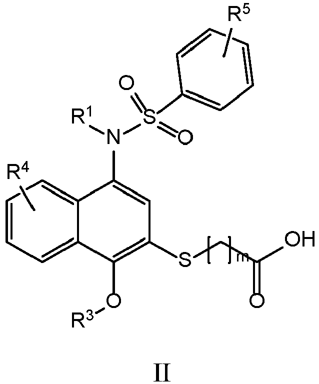
wherein, R³ is hydrogen, 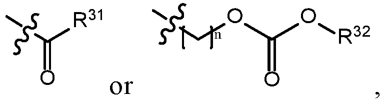 wherein, R³¹ and R³² are each independently C_{1~6} alkyl or C_{1~6} cycloalkyl, n is 1~4 ;
when R⁴ is not 0, each is independently selected from halogen, nitro, nitrile, -N⁺(R⁴⁻¹)₃, C_{1∼6} haloalkyl, -C(O)OR⁴⁻¹ , -C(O)R⁴⁻¹ , -C(O)N(R⁴⁻¹R^{4-1a}) , -S(O)₂R⁴⁻¹ , -S(O)R⁴⁻¹ , -N=C(R⁴⁻ R^{4-1a}) , wherein R⁴⁻¹ and R^{4-1a} are each independently hydrogen, C_{1~6} alkyl, C₂₋₆ alkenyl, C₂₋₆ alkynyl , C_{1~6} alkyl with at least one hydrogen substituted by halogen, C₂₋₆ alkenyl with at least one hydrogen substituted by halogen , or C₂₋₆ alkynyl with at least one hydrogen substituted by halogen ;
R⁵ is phenyl, naphthyl, 5- or 6-membered monocyclic heteroaryl, 8 to 10-membered fused bicyclic heteroaryl, phenyl with at least one hydrogen atom substituted by R⁵⁻¹ , naphthyl with at least one hydrogen atom substituted by R⁵⁻¹, 5- or 6-membered monocyclic heteroaryl with at least one hydrogen atom substituted by R⁵⁻¹ , 8- to 10-membered fused bicyclic heteroaryl with at least one hydrogen atom substituted by R⁵⁻¹, wherein R⁵⁻¹ is hydrogen, halogen, nitro, nitrile, hydroxyl , C_{1~6} alkyl, C_{3~6} cycloalkyl, C_{1~6} alkoxy , -N(R^{5 -1a}R^{5-1b} ), phenyl, C_{1~6} haloalkyl, C_{1~6} haloalkoxy, - C(O)OR⁵⁻¹¹, -C(O)R⁵⁻¹¹ , -C(O )N(R^{5-1a}R^{5-1b}), -S(O)₂R⁵⁻¹¹ , -S(O)R⁵⁻¹¹ , - OC(O)R⁵⁻¹¹, -OC(O)OR⁵⁻¹¹ or 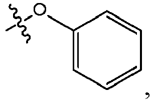 wherein R⁵⁻¹¹ , R^{5-1a} and R^{5-1b} are each independently hydrogen, C_{1~6} alkyl, three to six-membered cycloalkyl, C₂₋₆ alkenyl, C₂₋₆ alkynyl, C_{1~6} alkyl with at least one hydrogen substituted by halogen , C₂₋₆ alkenyl with at least one hydrogen substituted by halogen, C_{3~6} cycloalkyl with at least one hydrogen substituted by halogen , C₂₋₆ alkynyl with at least one hydrogen substituted by halogen;
R⁶ is -C(O)OR⁶⁻¹ , 5 or 6-membered monocyclic heteroaryl , wherein R⁶⁻¹ is hydrogen, C_{1~6} alkyl, three- to six-membered cycloalkyl, C₂₋₆ alkenyl, C₂₋₆ alkynyl, C_{1~6} alkyl with at least one hydrogen substituted by halogen , C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen ;
m is 1~6;
R¹ is C_{1~6} alkyl, three- to six-membered cycloalkyl, three- to six-membered epoxyalkyl, phenyl, C_{1~6} alkyl with at least one hydrogen substituted by R¹⁻¹ , phenyl with at least one hydrogen substituted by R¹⁻¹ , wherein R¹⁻¹ is hydroxyl, halogen, amino or C_{1~6} alkoxy.

Based on the fact that it is beneficial for the compound to improve its ability to selectively induce autophagy in damaged mitochondria, in some more preferred solutions, the compound has a structure represented by the formula (II) ,
wherein R³ is hydrogen, 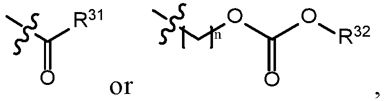 wherein R³¹ and R³² are independently C_{1~6} alkyl or C_{1~6} cycloalkyl, n is 1~4 ;
when the number of R⁴ is not 0, each is independently selected from halogen, nitro, nitrile , -N ⁺ (R⁴⁻¹)₃, C_{1∼6} haloalkyl, -C(O)OR⁴⁻¹ , -C(O)R⁴⁻¹ , -C(O)N(R⁴⁻¹R^{4-1a}) , -S(O)₂R⁴⁻¹ , -S(O)R⁴⁻¹ , -N=C(R⁴⁻¹R^{4-1a} ), wherein R⁴⁻¹ and R^{4-1a} are independently hydrogen, C_{1~6} alkyl, C₂₋₆ alkenyl , C₂₋₆ alkynyl , C_{1~6} alkyl with one hydrogen substituted by halogen, C₂₋₆ alkenyl with at least one hydrogen substituted by halogen , or C₂₋₆ alkynyl with at least one hydrogen substituted by halogen;
R⁵ is phenyl, naphthyl, 5- or 6-membered monocyclic heteroaryl, 8 to 10- membered fused bicyclic heteroaryl, phenyl with at least one hydrogen atom substituted by R⁵⁻¹ , naphthyl with at least one hydrogen atom substituted by R⁵⁻¹ , 5- or 6-membered monocyclic heteroaryl with at least one hydrogen atom substituted by R⁵⁻¹ , 8- to 10-membered fused bicyclic heteroaryl with at least one hydrogen atom substituted by R⁵⁻¹, where R⁵⁻¹ is hydrogen, halogen, nitro, nitrile, hydroxyl , C_{1~6} alkyl, C_{3~6} cycloalkyl, C_{1~6} alkoxy , -N(R^{5-1a}R^{5-1b}), phenyl, C_{1~6} haloalkyl, C_{1~6} haloalkoxy, - C(O)OR⁵⁻¹¹, -C(O)R⁵⁻¹¹ , -C(O )N(R^{5-1a}R^{5-1b}), -S(O)₂R⁵⁻¹¹ , -S(O)R⁵⁻¹¹ , - OC(O)R⁵⁻¹¹, -OC(O)OR⁵⁻¹¹ or 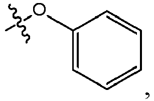 wherein R⁵⁻¹¹ , R^{5-1a} and R^{5-1b} are independently hydrogen, C_{1~6} alkyl, three to six-membered cycloalkyl, C₂₋₆ alkenyl, C₂₋₆ alkynyl , C_{1~6} alkyl with at least one hydrogen substituted by halogen, C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen;
R⁶ is -C(O)OR⁶⁻¹, 5- or 6-membered monocyclic heteroaryl , wherein R⁶⁻¹ is hydrogen, C_{1~6} alkyl, three- to six-membered cycloalkyl, C₂₋₆ alkenyl, C₂₋₆ alkynyl, C_{1~6} alkyl with at least one hydrogen substituted by halogen, C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen;
m is 1~6 .

Based on the fact that it is beneficial for the compound to improve its ability to selectively induce autophagy in damaged mitochondria, in some more preferred solutions, the compound is:

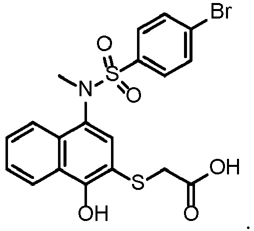

Furthermore, based on the fact that it is more beneficial to the enhancement of the compound's ability to induce mitophagy, in some preferred solutions, the compound has a structure represented by a formula (III); 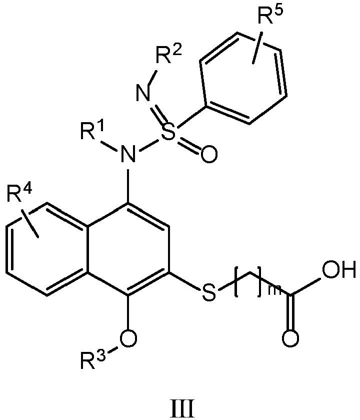
wherein R³ is hydrogen, 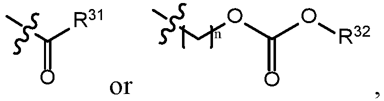 wherein R³¹ and R³² are independently C_{1~6} alkyl or C_{1~6} cycloalkyl, n is 1~4 ;
when the number of R⁴ is not 0, each is independently selected from halogen, nitro, nitrile , -N⁺(R⁴⁻¹)₃, C_{1∼6} haloalkyl, -C(O)OR⁴⁻¹, -C(O)R⁴⁻¹, - C(O)N(R⁴⁻¹R^{4-1a}) , -S(O)₂R⁴⁻¹ , -S(O)R⁴⁻¹ , -N=C (R⁴⁻¹R^{4-1a}) , wherein R⁴⁻¹ and R^{4-1a} are independently hydrogen, C_{1~6} alkyl, C₂₋₆ alkenyl , C₂₋₆ alkynyl, C_{1~6} alkyl with at least one hydrogen substituted by halogen, C₂₋₆ alkenyl with at least one hydrogen substituted by halogen , or C₂₋₆ alkynyl with at least one hydrogen substituted by halogen;
R⁵ is phenyl, naphthyl, 5- or 6-membered monocyclic heteroaryl, 8- to 10- membered fused bicyclic heteroaryl, phenyl with at least one hydrogen atom substituted by R⁵⁻¹ , naphthyl with at least one hydrogen atom substituted by R⁵⁻¹, 5- or 6-membered monocyclic heteroaryl with at least one hydrogen atom substituted by R⁵⁻¹ , 8- to 10-membered fused bicyclic heteroaryl with at least one hydrogen atom substituted by R⁵⁻¹ , wherein R⁵⁻¹ is hydrogen, halogen, nitro, nitrile, hydroxyl, C_{1~6} alkyl, C_{3~6} cycloalkyl, C_{1~6} alkoxy , -N(R^{5-1a}R^{5-1b}), phenyl, C_{1~6} haloalkyl, C_{1∼6} haloalkoxy, - C(O)OR⁵⁻¹¹, -C(O)R⁵⁻¹¹ , -C(O )N(R^{5-1a}R^{5-1b}), -S(O)₂R⁵⁻¹¹ , -S(O)R⁵⁻¹¹ , - OC(O)R⁵⁻¹¹, -OC(O)OR⁵⁻¹¹ or 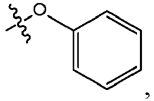 wherein R⁵⁻¹¹ , R^{5-1a} and R^{5-1b} are independently hydrogen, C_{1~6} alkyl, three- to six-membered cycloalkyl, C₂₋₆ alkenyl, C₂₋₆ alkynyl, C_{1~6} alkyl with at least one hydrogen substituted by halogen, C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen;
R⁶ is -C(O)OR⁶⁻¹, 5- or 6-membered monocyclic heteroaryl , wherein R⁶⁻¹ is hydrogen, C_{1~6} alkyl, three- to six-membered cycloalkyl, C₂₋₆ alkenyl, C₂₋₆ alkynyl, C_{1~6} alkyl with at least one hydrogen substituted by halogen , C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen ;
m is 1~6 ;
R¹ is hydrogen, C_{1~6} alkyl, three- to six-membered cycloalkyl, three-to six-membered epoxyalkyl, phenyl, C_{1∼6} alkyl with at least one hydrogen substituted by R¹⁻¹ , phenyl with at least one hydrogen substituted by R¹⁻² , wherein R¹⁻² is hydroxyl, halogen, amino or C_{1∼6} alkoxy ,
R² is a C_{1~6} alkyl, three- to six-membered cycloalkyl, three- to six-membered epoxyalkyl, or C_{1~6} alkyl in which at least one hydrogen is substituted by R²⁻¹ , wherein R²⁻¹ is hydroxyl, halogen, amino or C_{1∼6} alkoxy;
when R¹ and R² are independently C_{1~6} alkyl, R¹ and R² can be bonded to form a 4~8-membered ring.

In some preferred embodiments, in the formula (III) , R¹ is hydrogen, methyl, ethyl, cyclopropanyl or cyclobutanyl .

In some preferred embodiments, in the formula (III) , R² is hydrogen, methyl, ethyl, phenyl, cyclopropyl, cyclobutanyl or 2-hydroxyethyl.

In some preferred embodiments, in the formula (III) , when R¹ is methyl or ethyl and R¹ is methyl or ethyl, R¹ and R² can be bonded to form a 5- to 6-membered ring.

In some preferred embodiments, in the formula (III) , when the number of R⁴ is not 0, each is independently selected from bromine, nitro, and nitrile.

In some preferred embodiments, inthe formula (III) , R⁵ is phenyl or phenyl with at least one hydrogen atom substituted by bromine.

In a second aspect of the present invention, it provides a pharmaceutical composition comprising the compound as described in the first aspect of the present invention, and a pharmaceutically acceptable salt, stereoisomer, solvate or prodrug thereof.

In a third aspect of the present invention, it provides a use of the compound, or a pharmaceutically acceptable salt, stereoisomer, solvate or prodrug thereof described in the first aspect of the present invention, or the pharmaceutical composition described in the second aspect of the present invention for the preparation of a mitophagy inducer (especially a selective mitophagy inducer) .

Compared with the prior art, the present invention has at least the following advantages:
(1) The compound and the pharmaceutically acceptable salt, stereoisomer, solvate or prodrug thereof provided by the first aspect of the present invention or the pharmaceutical compositions provided by the second aspect of the present invention can enhance the ability to induce mitophagy compared to UMI-77;
(2) The compounds and the pharmaceutically acceptable salt, stereoisomer, solvate or prodrug thereof provided in the first aspect of the present invention or the pharmaceutical compositions provided in the second aspect of the present invention have superior metabolic stability and pharmacokinetic properties compared to UMI-77;
(3) The compounds and the pharmaceutically acceptable salt, stereoisomer, solvate or prodrug thereof provided by the first aspect of the present invention or the pharmaceutical compositions provided by the second aspect of the present invention are less toxic and have better drug-forming properties compared to UMI-77;
(4) The compound and the pharmaceutically acceptable salt, stereoisomer, solvate or prodrug thereof provided in the first aspect of the present invention or the pharmaceutical composition provided in the second aspect of the present invention.

Therefore, the compounds of the present invention have the effect of inducing autophagy in damaged mitochondria or improving metabolic stability. Preferred compounds of the present invention have the effect of both inducing autophagy in damaged mitochondria and improving metabolic stability.

More preferably, preferred compounds of the present invention have the effect of selectively inducing autophagy in damaged mitochondria or improving metabolic stability. Particularly preferred compounds of the present invention have the effect of both selectively inducing autophagy in damaged mitochondria and improving metabolic stability.

It should be understood that within the scope of the present invention, the above-mentioned technical features of the present invention and the technical features specifically described below (such as embodiments) can be combined with each other to form new or preferred technical solutions. Due to space limitations, they will not be repeatedly described one by one herein.

### Description of the drawings

One or more embodiments are exemplified by the pictures in the corresponding drawings, and these exemplary illustrations do not constitute limitations to the embodiments.
Fig.1 is a diagram showing the results of inducing mitophagy in human embryonic kidney transformed cells HEK293T-mtkeima system directly treated with **UMI-77** or compound **I-4 in** Test Example 1 of the present invention;
Fig.2 is a diagram showing the experimental results of selective induction of mitophagy **by UMI-77** or compound **I-1** in Test Example 1 of the present invention;
Fig.3 is a diagram showing the results of the in vitro liver microsome stability experiment of **UMI-77** and Compound **I-4** in Test Example 2 of the present invention.

### Detailed description

In the study, the inventors have found that UMI-77 has a relatively weak ability to induce mitophagy, and its metabolism is unstable and its druggability is poor. In order to solve this problem, the inventors of the present invention have conducted a large number of experimental studies and found that the compound as described in the first aspect of the present invention is prepared by modifying part of the structure of UMI-77, which can significantly enhance the ability to induce autophagy in mitochondria, and at the same time improve the druggability.

Preferably, the inventors have found that on the basis of UMI-77 , modification of the hydrogen on the imine group 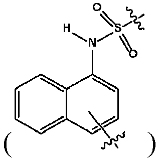 directly attached to the naphthalene ring, such as alkylation ("H" is substituted by C₁₋₆ alkyl or three- to six-membered cycloalkyl) described in some embodiments of the present invention, or when "H" is substituted by the R¹ group described in some embodiments of the present invention , such as three to six-membered epoxyalkyl, phenyl substitution, allows the compounds to embody the ability to partially selectively induce autophagy in damaged mitochondria.

Preferably, the inventor has found that on the basis of UMI-77 , the ability of the compound to induce mitophagy can be further enhanced by modifying any of the oxygens on the group 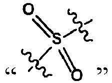 to "NH" or to " N-R² " as described in some embodiments of the present invention.

Preferably, the inventors have found that modifying the phenolic hydroxyl on the naphthalene ring as described in some embodiments of the present invention, such as esterification modification, is beneficial to shield ionizable sites and enhance transmembrane absorption.

### Terms

As used herein, the term "mitophagy" refers to a process of selective degradation of mitochondria by autophagy, which is an important mechanism for the control of mitochondrial quality and quantity.

As used herein, the term " mitophagy inducer" refers to a compound that can induce mitophagy function.

As used herein, the term "alkyl" refers to a straight-chain or branched saturated aliphatic hydrocarbon group. The term "C₁₋₆ alkyl" refers to a straight-chain or branched alkyl having 1 to 6 carbon atoms, non-limiting examples are: methyl, ethyl, n-propyl , isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers, etc. The term "C₁₋₄ alkyl" refers to a straight-chain or branched alkyl having 1 to 4 carbon atoms. If the C₁₋₄ alkyl appears at the end of the molecule, non-limiting examples are: methyl, ethyl, n-propyl, isopropyl, n-butyl, isobutyl, sec-butyl, tert-butyl, or when two parts of the molecule are connected through the alkyl, non-limiting examples are: -CH₂- , -CH₂-CH₂-, -CH(CH₃)- , -CH₂-CH₂-CH₂-, - CH(C₂H₅)-, -C(CH₃)₂-, each hydrogen of the C₁₋₄ alkyl may be substituted by a substituent further enumerated herein.

As used herein, the term "alkenyl" refers to a straight or branched hydrocarbon chain containing at least one carbon-carbon double bond. Each hydrogen in the alkenyl carbon may be substituted by a substituent further enumerated herein. The term "C_{2∼6} alkenyl" refers to a straight or branched hydrocarbon chain of 1 to 6 carbon atoms containing at least one carbon-carbon double bond. If it appears at the end of the molecule, non-limiting examples are: -CH=CH₂, -CH=CH-CH₃, -CH₂-CH=CH₂, - CH=CH-CH₂ -CH₃, -CH=CH-CH=CH₂, or when two parts of the molecule are connected through the alkenyl, a non-limiting example is - CH=CH-. Each hydrogen of the C_{2~6} alkenyl carbons may be substituted by a substituent further enumerated herein.

As used herein, the term "alkynyl" refers to a straight or branched hydrocarbon chain containing at least one carbon-carbon triple bond. Each hydrogen in the alkynyl carbon may be substituted by a substituent further enumerated herein. The term "C_{2∼6} alkynyl " refers to a straight or branched hydrocarbon chain having 1 to 6 carbon atoms containing at least one carbon-carbon triple bond. If it appears at the end of the molecule, non-limiting examples are: -C=CH, -CH₂-C≡CH, -CH₂-CH₂ -C=CH, -CH₂-C≡C-CH₃ , or , when two parts of the molecule are connected through the alkynyl, a non-limiting example is -C=C-. Each hydrogen in the C_{2~6} alkynyl carbon may be substituted by a substituent further enumerated herein.

As used herein, the term "alkoxy" refers to a group having the structure "-O-alkyl", wherein the alkyl is as defined above. The term "C_{1~6} alkoxy" refers to an alkoxy group having 1 to 6 carbon atoms , non-limiting examples are: methoxy, ethoxy, n-propoxy , isopropoxy, n-butoxy , tert-butoxy , isobutoxy , n- pentyloxy, etc.

As used herein, the term "amido" refers to a group formed by substituting at least one hydrogen atom in the amino with an alkyl. For example: in the amido 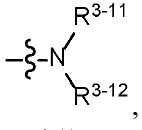 either one of R³⁻¹¹ and R³⁻¹² is alkyl, the other is hydrogen; or R³⁻¹¹ and R³⁻¹² are all alkyl; when R³⁻¹¹ and R³⁻¹² are all alkyl, R³⁻¹¹ and R³⁻¹² can be bonded to form a ring.

As used herein, the term "haloalkyl" refers to an alkyl with one or more (e.g., 1, 2, 3, 4 or 5 ) hydrogen atoms substituted by halogen, wherein the alkyl is as defined above.

As used herein, "(O)" refers to 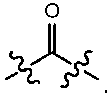 In one embodiment, -CH₂ C(O)R³⁻² refers to

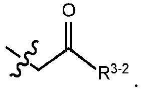

As used herein, the term "halooxyalkyl" refers to an alkoxy with one or more hydrogen atoms substituted by halogen, wherein the alkoxy is as defined above.

As used herein, the terms "aryl", "aryl ring" and "aromatic ring" are used interchangeably and refer to all-carbon monocyclic, all-carbon non-fused polycyclic (rings connected by covalent bonds, non-fused ) or all-carbon fused polycyclic (that is, rings sharing pairs of adjacent carbon atoms) groups, at least one ring in the group is aromatic, that is, it has a ring-forming conjugated π electron system.

As used herein, the term "heteroaryl" refers to an aryl group in which at least one of the ring carbon atoms making up the aryl is replaced by a heteroatom that is a non-carbon atom , such as S, N, or O.

As used herein, the term "monocyclic heteroaryl" refers to a heteroaryl group having only one aromatic ring, wherein heteroaryl is as defined above. The term "5- or 6-membered monocyclic heteroaryl" refers to a a monocyclic heteroaryl having 5 or 6 ring atoms, of which 1, 2 or 3 ring atoms are heteroatoms selected from nitrogen, oxygen or S(=O)m' (wherein m' is an integer 0 to 2), non-limiting examples are: thiophene, furan, thiazole, isothiazole, imidazole, oxazole, pyrrole , pyrazole , triazole ( e.g. 1, 2, 3-triazole , 1,2,4-triazole , 1,2,5-triazole, 1,3,4-triazole, etc. ), tetrazole , isoxazole , oxadiazole, 1,2,3-oxadiazole, 1,2,4-oxadiazole , 1,2,5-oxadiazole, 1,3,4 oxadiazole, thiadiazole , pyridine , pyridazine , pyrimidine, pyrazine etc.

As used herein, the term "fused ring heteroaryl" refers to having at least two aromatic rings in which two ring atoms are adjacent to each other, wherein heteroaryl is as defined above. The term "fused bicyclic heteroaryl" refers to a fused ring heteroaryl having two aromatic rings, wherein the fused ring heteroaryl is as defined above. The term "8 to 10-membered fused bicyclic heteroaryl" refers to those having 8 to 10 ring atoms, of which 1, 2, 3, 4 or 5 ring atoms are heteroatoms selected from nitrogen, oxygen, or S(=O)m' (wherein m' is an integer 0 to 2), non-limiting examples include: benzo[d] isoxazole , 1H-indole, isoindole, 1H-benzo[d]imidazole, benzo[d]isothiazole, 1H-benzo[d][1,2,3]triazole , benzo[d] oxazole , benzo[d]thiazole, indazole , benzofuran, benzo[b] thiophene, quinoline, isoquinoline, quinazoline, quinoxaline , cinnoline , pyrido[3,2-d]pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine, etc.

As used herein, the term "effective dose" or "therapeutically effective dose" refers to a chemical entity (e.g., a compound that exhibits activity as a modulator of NLRP1/3, or a pharmaceutically acceptable salt and/or a hydrate and/or a cocrystal thereof;) in a sufficient amount that, when administered, the dose will alleviate to a certain extent one or more of the symptoms of the disease or condition being treated. Results include alleviation and/or remission of signs, symptoms or causes of disease or any other desired change in a biological system. The appropriate "effective" dose in any individual situation is determined using any appropriate technique, such as dose escalation studies.

As used herein, the term "excipient" or "pharmaceutically acceptable excipient" means a pharmaceutically acceptable material, composition or vehicle such as a liquid or solid filler, diluent, carrier, solvent, or packaging materials. In one embodiment, each component is compatible with the other ingredients of the pharmaceutical formulation and is suitable for contacting with human and animal tissues or organs without undue toxicity, irritation, allergic response, immunogenicity or other problems or complications and is "pharmaceutically acceptable" in the sense of being proportionate to a reasonable benefit/risk ratio.

As used herein, the term "pharmaceutically acceptable salts" may refer to pharmaceutically acceptable addition salts prepared from pharmaceutically acceptable non-toxic acids, including inorganic acids and organic acids. In some cases, pharmaceutically acceptable salts are obtained by reacting a compound described herein with an acid. The term "pharmaceutically acceptable salt" may also refer to the formation of a salt by reacting a compound having an acidic group with a base or pharmaceutically acceptable addition salts prepared by other methods as previously determined. The pharmacologically acceptable salt is not particularly limited as long as it can be used in medicines. Examples of salts formed by the compounds described herein with bases include the following : salts with inorganic bases such as sodium, potassium, magnesium, calcium and aluminium; salts with organic bases such as methylamine , ethylamine, and ethanolamine; or formed by reaction with dicyclohexylamine, N-methyl-D-glucosamine or tris(hydroxymethyl)methylamine; salts with basic amino acids such as lysine and ornithine; and ammonium salts. The salt may be an acid addition salt, this is exemplified by the addition of salts to the following acids: inorganic acids, such as hydrochloric acid, hydrobromic acid, hydroiodic acid, sulfuric acid, nitric acid and phosphoric acid; organic acids, such as formic acid, acetic acid, propionic acid, oxalic acid, malonic acid, succinic acid, fumaric acid, maleic acid, lactic acid, malic acid, tartaric acid, citric acid, methane sulfonic acid and ethanesulfonic acid; acidic amino acids such as aspartic acid and glutamic acid .

As used herein, the term "excipient" or "pharmaceutically acceptable excipient" means a pharmaceutically acceptable material, composition, or vehicle, such as a liquid or solid filler, diluent, carrier, solvent, or encapsulating material. In one embodiment, each component is compatible with the other ingredients of the pharmaceutical formulation and is suitable for contacting with human and animal tissues or organs without undue toxicity, irritation, allergic response, immunogenicity or other problems or complications, and is "pharmaceutically acceptable" in the sense of being proportionate to a reasonable benefit/risk ratio.

As used herein, the term "pharmaceutical composition" refers to a mixture of a compound described herein and "excipients" such as carriers, stabilizers, diluents, dispersing agents, suspending agents and/or thickening agents. Pharmaceutical compositions facilitate the administration of compounds to an organism. A variety of techniques for administering compounds exist in the art, including, but not limited to, rectal, oral, intravenous, aerosol, parenteral, ocular, pulmonary, and topical administration.

As used herein, the term "subject" refers to an animal, including, but not limited to, a primate (e.g., a human), a monkey, a cow, a pig, a sheep, a goat, a horse, a dog, a cat, a rabbit, a rat, or a mouse. The terms "subject" and "patient" are used interchangeably herein, for example with respect to mammalian subjects (e.g., humans).

As used herein, the terms "treat, treating and treatment" in the context of treating a disease or disorder are meant to include alleviation or elimination of the disorder, disease or condition, wherein the term "disorder" as used herein shall always be understood to mean "a disorder, disease or condition" or one or more of the symptoms associated with a disorder; or slowing a disorder or condition or slowing the progression, spread or worsening of one or more of its symptoms.

Unless otherwise specified, as used herein, 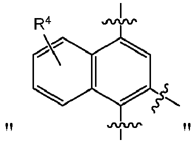 means that the naphthalene ring (including the two benzene rings on the left and the right) is substituted by 0 , 1, 2, 3, 4 or 5 R⁴, and when the naphthalene ring is substituted by more than 1 of R⁴, each R⁴ is the same or different.

Unless otherwise specified, "selectively" as used herein refers to the property of significantly inducing damaged mitophagy without affecting normal mitochondria or only weakly affecting normal mitochondria . "Metabolic stability" as used herein includes, but is not limited to, liver microsome stability and plasma stability.

In order to make the purpose, technical solutions and advantages of the embodiments of the present invention clearer, the present invention will be further described below in conjunction with specific embodiments. It should be understood that these embodiments are only used to illustrate the invention and are not intended to limit the scope of the invention. Experimental methods without specifying specific conditions in the following examples usually follow conventional conditions or conditions recommended by the manufacturer. Unless otherwise stated, percentages and parts are by weight. The experimental materials and reagents used in the following examples can be obtained from commercial sources unless otherwise specified.

Unless otherwise specified, technical and scientific terms used herein have the same meanings as commonly understood by those of ordinary skill in the technical field to which this application belongs. It should be noted that the terms used herein are only for describing specific embodiments and are not intended to limit the exemplary embodiments of the present invention.

### Embodiment 1 : Synthesis and characterization of compound I-1

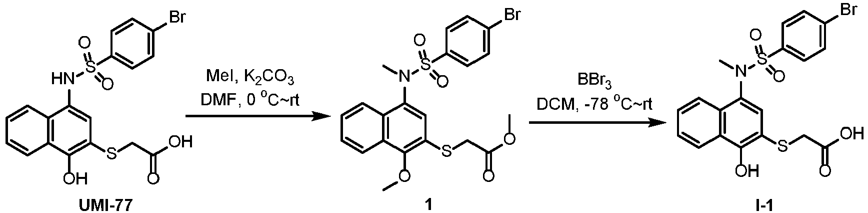

Dissolving **UMI -77** ( 45 mg, 0.096mmol) in DMF (0.5 mL) under nitrogen atmosphere and stirring in an ice-water bath, adding K₂ CO₃ ( 39 mg, 0.28mmol ) and MeI (30 µL, 0.48 mmol ) in sequence, keeping stirring in the ice-water bath for 10 min, then returning to room temperature and continuing stirring overnight. Detecting the forming of trimethylation product 1 by LC-MS. Diluting the reaction system with DCM and performing silica gel column chromatography ( 30 % v/v ethyl acetate/petroleum ether system), thus obtaining compound 1 ( 42 mg) .

Dissolving Compound **1** (4.2 mg , 0.081 mmol) in dry DCM (0.5 mL ) and stirring in a dry ice-ethanol bath, adding a DCM solution of 1M BBr₃ (0.24 mL, 0.24 mmol) dropwise, then slowly returning to room temperature and continuing stirring for 2 hours. Detecting the product **I-1** by LC-MS. adding diluted hydrochloric acid to quench the reaction, extracting with DCM, and separating out an organic phase and drying over anhydrous sodium sulfate. Concentrating the organic phase, and purifying the obtained crude product by preparative HPLC ( MeCN /H ₂ O/TFA ) to obtain product **I-1** (23 mg).

Taking the purified product **I-1** for a structural characterization, LC-MS (ESI) m/z: 480, 482 [M-H]⁻. ¹H-NMR (400 MHz, DMSO-*d₆*) δ (ppm): 12.90 (s, 1H), 10.08 (s, 1H), 8.22 (d, J = 8.2 Hz, 1H), 8.05 (d, J = 8.2 Hz, 1H), 7.88 (d, J = 8.1 Hz, 2H), 7.76 - 7.53 (m, 4H), 6.85 (s, 1H), 3.54 (s, 2H), 3.19 (s, 3H).

### Embodiment 2 : Synthesis and characterization of compound I-2

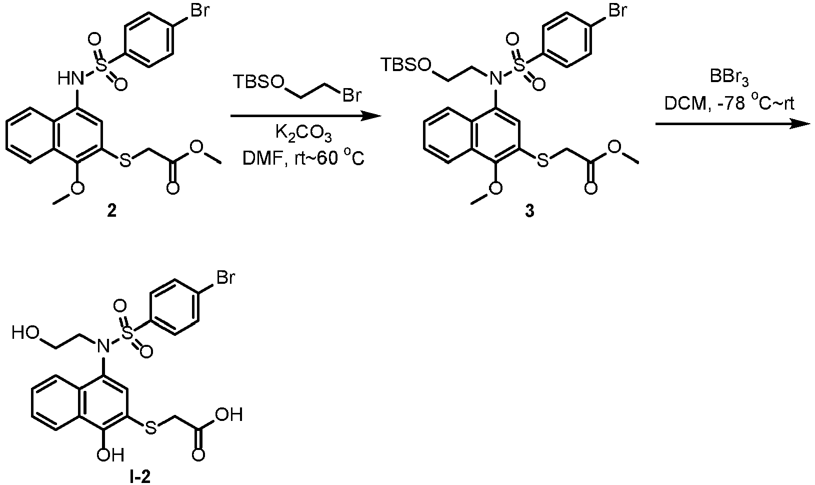

Using the known intermediate 2 ( J. Med. Chem. 2014, 57, 4111-4133 ) as raw material, preparing the compound I-2 according to the synthesis route of the compound **I-1**.

Taking the purified product **I-2** for a structural characterization, LC-MS (ESI) m/z: 510 , 512 [M - H] ⁻.

### Embodiment 3: Synthesis and characterization of compound I- 3

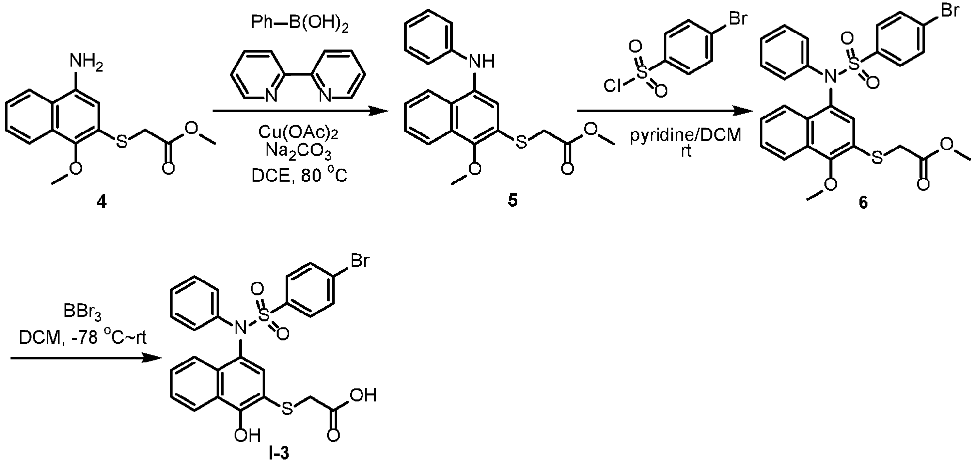

Dissolving a known compound **4** ( J. Med. Chem. 2014, 57, 4111-4133 ) ( 1.0mmol) and 2, 2'- dipyridyl (0.8 mmol) in dry DCE ( 8 mL ) , and adding phenylboronic acid (2.0 mmol), copper acetate (1.0 mmol) and sodium carbonate (2.0 mmol) sequentially and heating at 80 degrees Celsius and stirred for 24 hours. Detecting the product **5** by LC-MS. Purifying by silica gel column chromatography to obtain an intermediate **5** (98 mg). Dissolving the intermediate **5** ( 0.28 mmol) in DCM (0.4 mL ) and pyridine (0.3 mL ) and stirring in an ice-water bath, adding p-bromobenzenesulfonyl chloride (0.3 mmol ) , and then slowly returning to room temperature. Detecting the production of intermediate **6 by** LC-MS. Purifying by Silica gel column chromatography to obtain an intermediate **6** ( 79 mg) .

Using the intermediate **6** (0.14 mmol ) as raw material, preparing the compound **I-3** ( 23 mg) according to the last step of the synthesis route of the compound **I-2** .

Taking the purified product **I-3** for a structural characterization, LC-MS (ESI) m/z: 542, 544 [M - H] ⁻.

### Embodiment 4 : Synthesis and characterization of compound I- 4

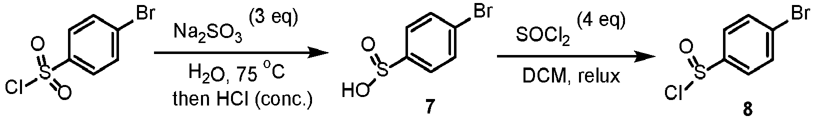

Dissolving Na₂SO₃ (3.8 g , 30 mmol ) in 30 mL deionized water, and adding p-bromobenzenesulfonyl chloride (2.6 g , 10 mmol ) in batches while stirring. Heating in an oil bath at 75°C for 5 hours, adding concentrated hydrochloric acid dropwise after cooling , precipitating a white solid out. After suction filtration, recrystallizing the obtained solid in water to obtain an intermediate 7 (1.3 g ). Suspending the intermediate 7 in 80 mL DCM, adding thionyl chloride (4 eq ) dropwise. After the complete of adding, heating in an oil bath to reflux, after 5 hours, removing the solvent by rotary evaporation, and taking the residual thionyl chloride out with toluene to obtain an intermediate 8 (626 mg) .

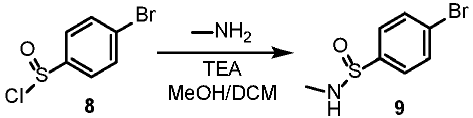

Suspending the intermediate 8 (4 8 mg, 0 .2 mmol) in 1 mL DCM, adding the alcohol solution of methylamine (1.5 eq) while cooling in an ice-water bath, then adding triethylamine (2 eq), and returning to room temperature naturally, and stirring for 2 hours. LC-MS shows the formation of an intermediate 9 . Purifying the intermediate 9 by silica gel column chromatography (EA in hexane = 65% v/v ) to obtain a light yellow solid (30 mg ).

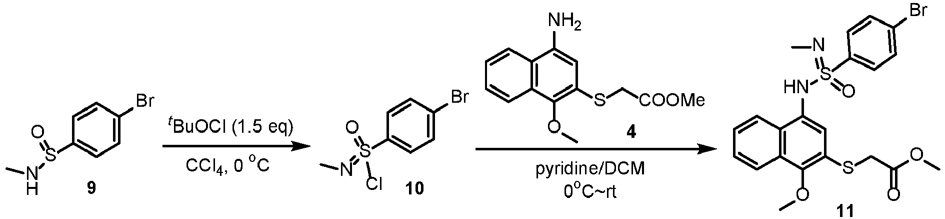

Suspending the intermediate 9 (30 mg , 0.13 mmol) in 0.5 mL carbon tetrachloride, stirring in an ice-water bath, adding tert-butyloxychloride (1.5 eq), and the solid is dissolved immediately. Continuing stirring for 1 hour and removing low-boiling substances by rotary evaporation to obtain an intermediate 10. Then dissolving the intermediate 10 in 0.5 mL DCM, stirring in an ice water bath. Dissolving an intermediate **4** ( 1 eq , prepared with reference to J. Med. Chem. 2014, 57, 4111-4133 ) in 0.3 mL pyridine, and adding this pyridine solution dropwise to the DCM solution of the intermediate **10** , returning to room temperature naturally and stirring overnight. LC-MS shows the formation of intermediate **11** . Purifying by silica gel column chromatography (EA in hexane = 40% v/v ) to obtain an intermediate **11** as oil matter (51 mg).

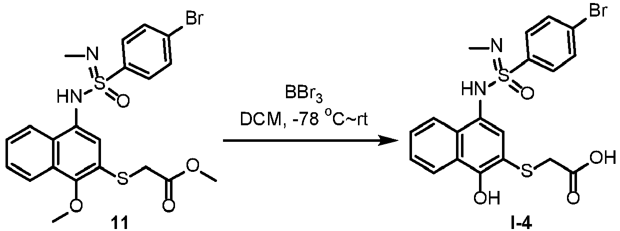

Dissolving the intermediate **11** (5 1 mg, 0 .1 mmol) in 0 .5 mL DCM, adding in a DCM solution of BBr₃ (0.2 mL , 1 M in DCM ) dropwise while cooling in a dry ice-ethanol bath. Slowly returning to room temperature and stirring for 3 hours, LC-MS shows that the intermediate **11** is completely consumed and compound **I-4** is generated. adding deionized water dropwise to quenching the reaction, and extracting three times with DCM. Combining the organic phases, evaporating the organic solvent by rotary evaporation , and purifying by preparative HPLC ( MeCN/H₂O/TFA ) to obtain a product **I-4** (13 mg).

Taking the purified product **I-4** for a structural characterization , LC-MS (ESI) m/z: 479 , 481 [M - H] ⁻. ¹H-NMR (400 MHz, DMSO- *d ₆*) δ (ppm): 9.13 (s, 1H), 8.36 - 8.28 (m, 1H), 8.14 - 8.06 (m, 1H), 7.93 (d, J = 8.4 Hz, 2H), 7.83 (d, J = 8.4 Hz, 2H), 7.55 - 7.39 (m, 3H), 7.22 (s, 1H), 3.61 (s, 2H), 2.41 (s, 3H).

### Embodiment 5 : Synthesis and characterization of compound I-5

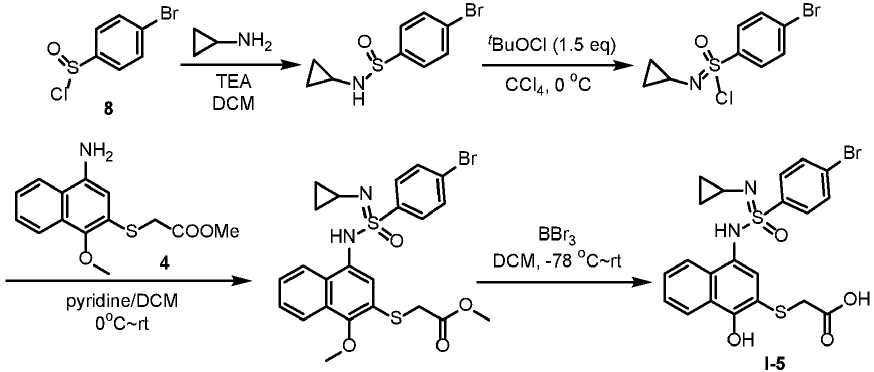

According to the synthesis route of the compound **I-4**, preparing the compound **I-5** by replacing methylamine therein with cyclopropylamine.

Taking the purified product **I-5** for a structural characterization, LC-MS (ESI) m/z: 505, 507 [M - H] ⁻. ¹ H-NMR (400 MHz, DMSO- *d ₆*) δ (ppm): 12.01 (s, 1H), 8.38 - 8.30 (m, 1H), 8.16 - 8.07 (m, 1H), 7.97 (d, J = 8.7 Hz, 2H), 7.84 (d, J = 8.6 Hz, 3H), 7.48 ( qd, J = 6.8, 3.4 Hz, 2H), 7.23 (s, 1H), 3.52 (s, 2H), 2.17 - 2.09 (m , 1H), 0.47 - 0.38 (m, 1H), 0.38 - 0.23 (m, 2H), 0.21 - 0.11 (m, 1H).

### Embodiment 6 : Synthesis and characterization of compound I-6

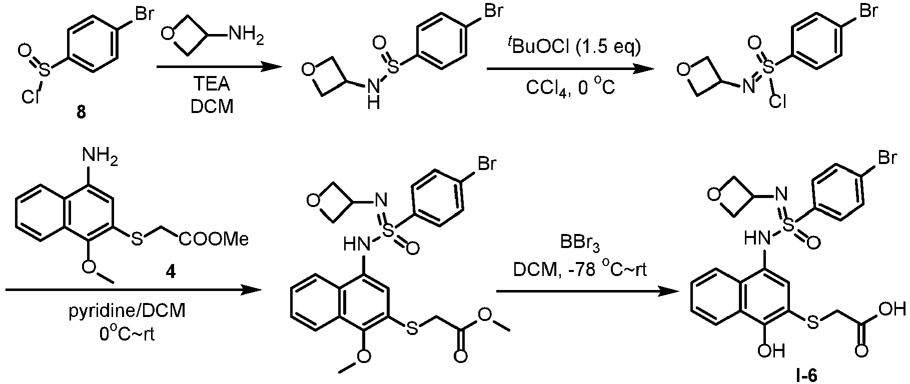

According to the synthesis route of the compound **I-4**, preparing the compound I-6 by replacing the methylamine therein with

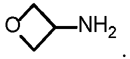

Taking the purified product **I-6** for a structural characterization, LC-MS (ESI) m/z: 521, 523 [M - H] ⁻ .

### Embodiment 7 : Synthesis and characterization of compound I-7

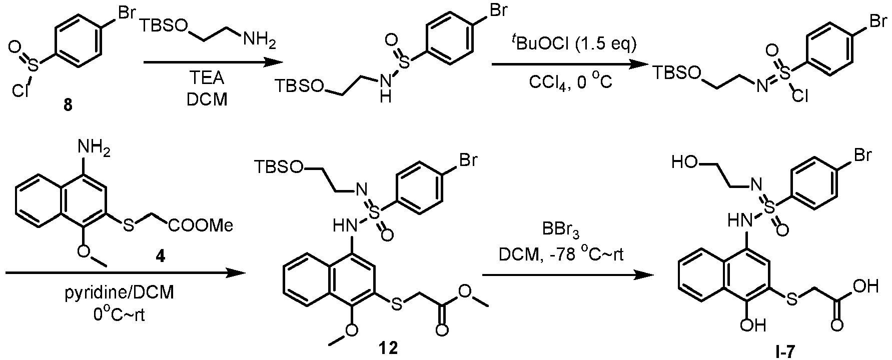

According to the synthesis route of the compound **I-4**, preparing the compound **I-7** by replacing the methylamine therein with

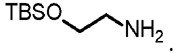

Taking the purified product **I**- 7 for a structural characterization, LC-MS (ESI) m/z: 509 , 511 [M - H] ⁻ .

### Embodiment 8 : Synthesis and characterization of compound I-8

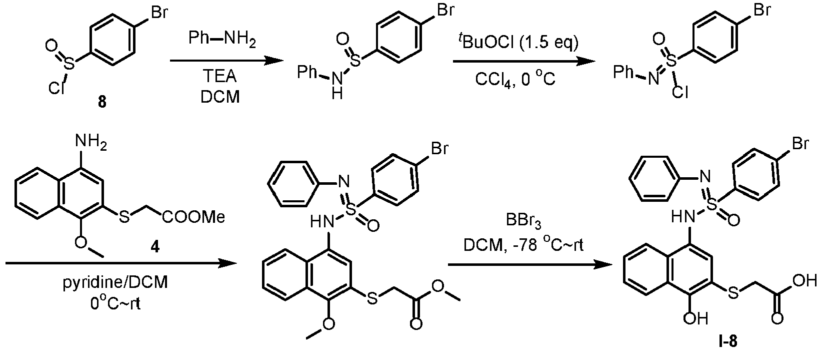

According to the synthesis route of the compound **I-4**, preparing the compound **I-8** by replacing methylamine therein with aniline.

Taking the purified product **I-8** for a structural characterization, LC-MS (ESI) m/z: 541, 543 [M - H] ⁻.

### Embodiment 9 : Synthesis and characterization of compound I-9

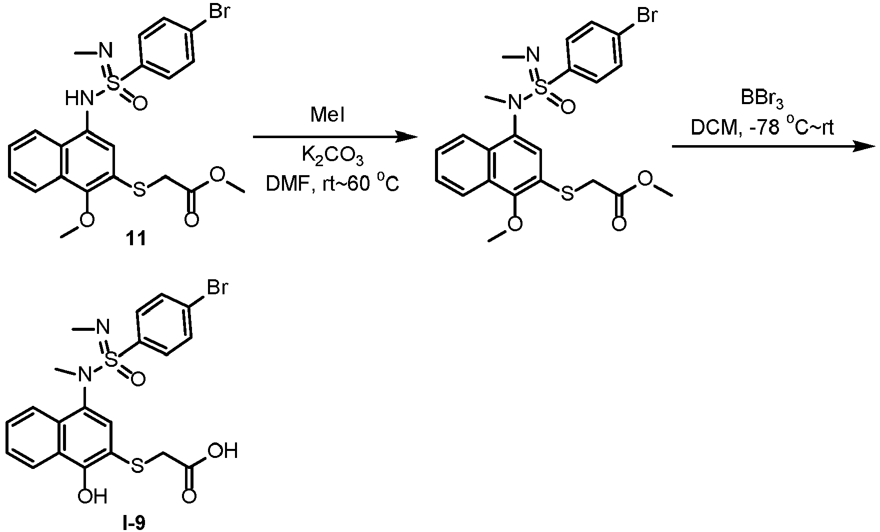

According to the synthesis route of the compound **I-2**, using the above-mentioned intermediate 11 as a raw material, preparing the compound **I-9**.

Taking the purified product **I-9** for a structural characterization , LC-MS (ESI) m/z: 493, 495 [M - H] ⁻.

### Embodiment 10 : Synthesis and characterization of compound I-10

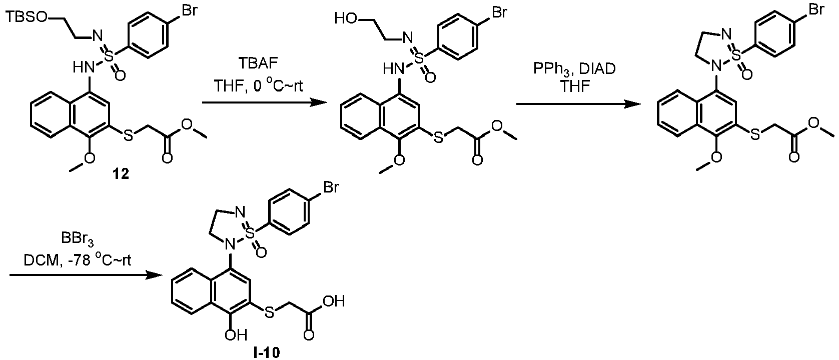

Dissolving an intermediate 12 (1.9 mmol) in THF, stirring at room temperature and adding a THF solution of 1 M TBAF dropwise, and continuing stirring for one hour until the TBS is completely removed. Obtaining an TBS-removed product (1.8 mmol) by silica gel column chromatography. Dissolving the TBS-removed product (1.8 mmol ) and triphenylphosphine (1.2 eq ) in anhydrous THF, stirring at room temperature and adding DIAD (1.2 eq) dropwise, continuing stirring at room temperature overnight, LC- MS shows that a cyclization product is produced, and performing silica gel column chromatography to obtain a cyclization product ( 0.8 mmol ).

Using the cyclization product as raw material, according to the last step of the synthesis route of compound **I-2**, and performing demethylation with boron tribromide to prepare the compound **I-10**.

Taking the purified product **I-10** for a structural characterization, LC-MS (ESI) m/z: 491, 493 [M - H] ⁻.

### Embodiment 11 : Synthesis and characterization of compound I- 11

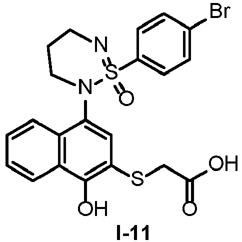

According to the synthesis method of compounds **I-7** and **I-10**, preparing the compound **I-11** by replacing 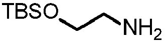 therein with

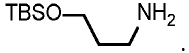

Taking the purified product **I- 11** for a structural characterization, LC-MS (ESI) m/z: 505, 507 [M - H] ⁻ .

### Embodiment 12 : Synthesis and characterization of compound I- 12

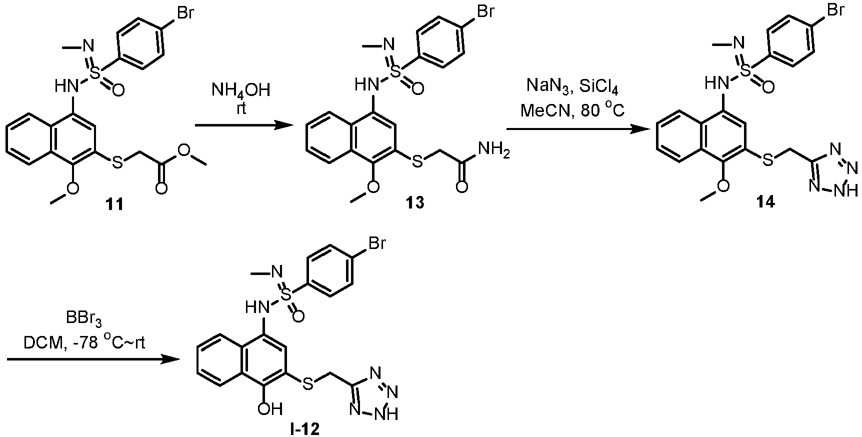

Using the above intermediate 11 as raw material, preparing the compound **I-12** according to the synthetic route in the above figure.

Taking the purified product 1- 12 for a structural characterization , LC-MS (ESI) m/z: 505, 507 [M + H]⁺ .

### Embodiment 13 : Synthesis and characterization of compound I- 13

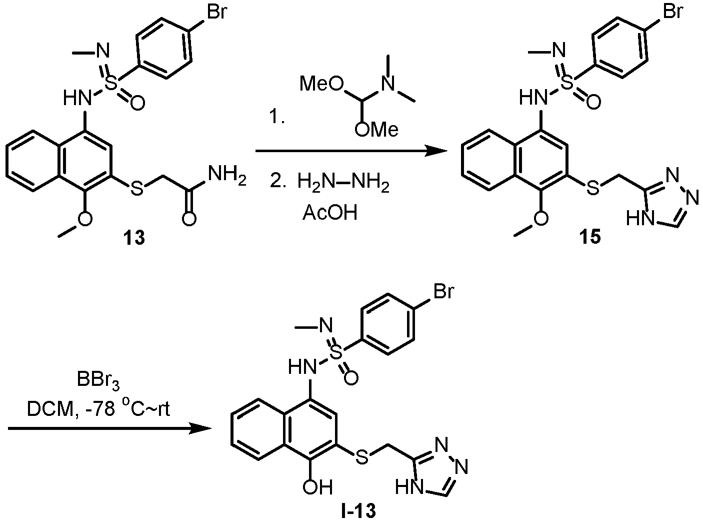

Using the above intermediate 13 as raw material, preparing the compound **I-13** according to the synthetic route in the above figure .

Taking the purified product **I-13** for a structural characterization , LC-MS (ESI) m/z: 504, 506 [M + H] + .

### Embodiment 14 : Synthesis and characterization of compound I-14

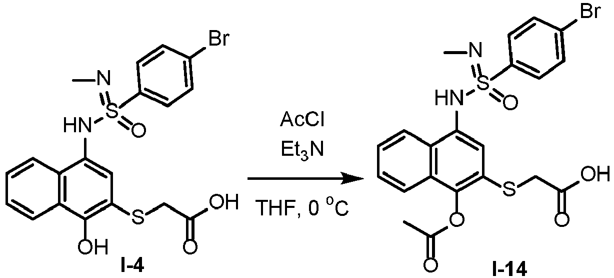

Preparing the compound **I-14** by using compound **I-4** as raw material and acylating the phenolic hydroxyl group with acetyl chloride.

Taking the purified product **I-14** for a structural characterization , LC-MS (ESI) m/z: 523, 525 [M + H] ⁺ .

### Embodiment 15 : Synthesis and characterization of compound I-15

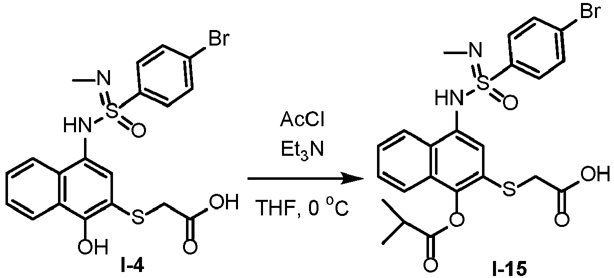

Using compound **I-4** as raw material according to the synthesis method of compound **I-14** , preparing the compound **I-15** by replacing acetyl chloride therein with

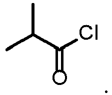

Taking the purified product **I-15** for a structural characterization, LC-MS (ESI) m/z: 551 , 553 [M + H] + .

### Embodiment 16 : Synthesis and characterization of compound I-16

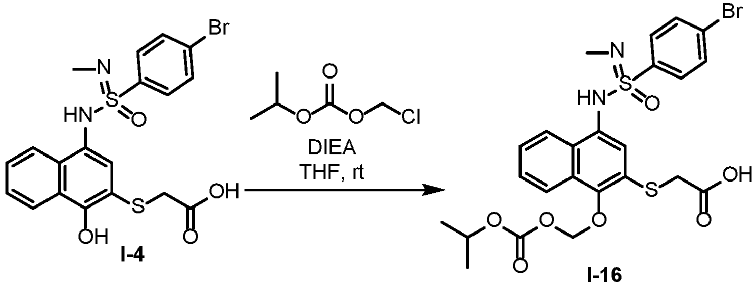

Using compound **I-4** as raw material, preparing the compound **I-16** through a one-step alkylation reaction .

Taking the purified product **I-16** for a structural characterization, LC-MS (ESI) m/z: 597, 599 [M + H] ⁺.

### Embodiment 17 : Synthesis and characterization of compound I-17

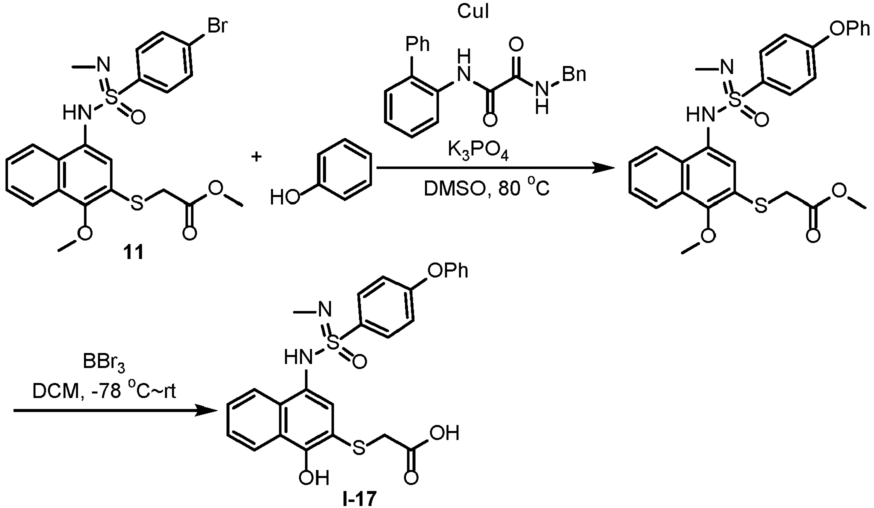

Using the above intermediate 11 as raw material ,preparing the Compound **I-17** according to the synthetic route in the above figure.

Taking the purified product **I-17** for a structural characterization , LC-MS (ESI) m/z: 493 [M - H] ⁻.

### Embodiment 18 : Synthesis and characterization of compound I-18

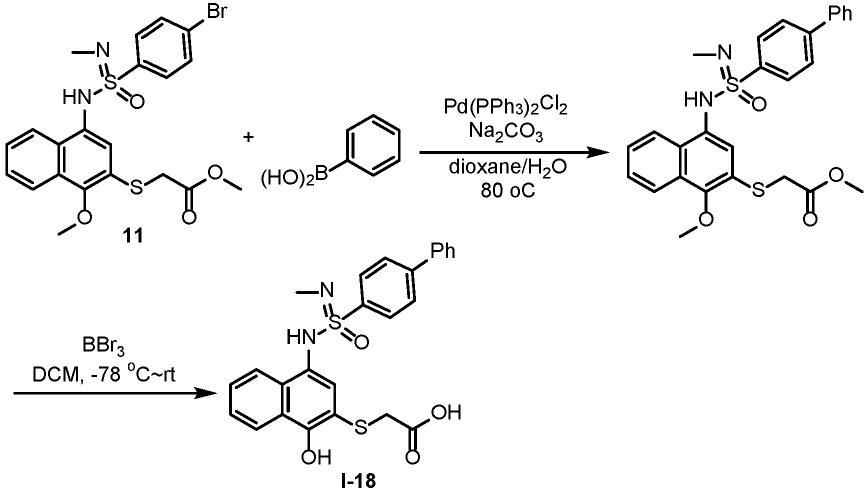

Using the above intermediate **11** as raw material, ,preparing the Compound **I-18** according to the synthetic route in the above figure .

Taking the purified product **I-18** for a structural characterization, LC-MS (ESI) m/z: 477 [M - H] ⁻.

### Embodiments 19 ~ 22 : Synthesis and characterization of compounds I-19 ∼ I-22

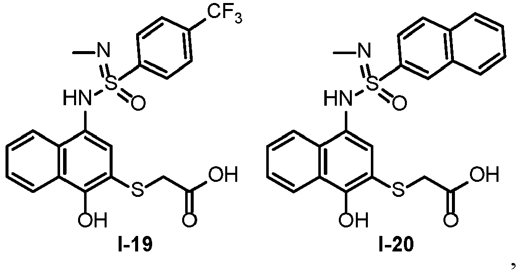

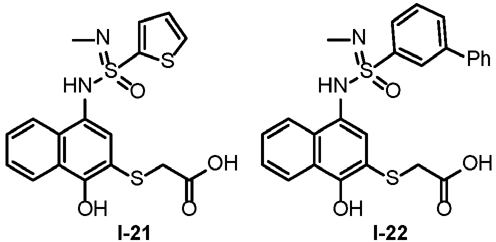

Preparing the compound **I-19** ~ **I-22** according to the synthesis route of the compound **I-4** by replacing 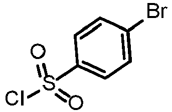 therein with 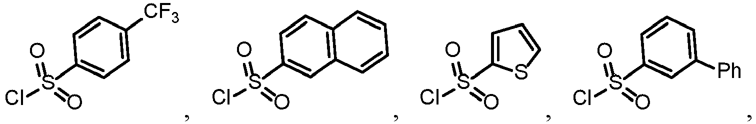 respectively.

Taking the purified product **I-19** for a structural characterization , LC-MS (ESI) m/z: 469 [M - H] ⁻.

Taking the purified product **I-20** for a structural characterization , LC-MS (ESI) m/z: 451 [M - H] ⁻.

Taking the purified product **I-21** for a structural characterization , LC-MS (ESI) m/z: 407 [M - H] ⁻.

Taking the purified product **I-22** for a structural characterization , LC-MS (ESI) m/z: 477 [M - H] ⁻.

### Embodiment 23 : Synthesis and characterization of compound I-23

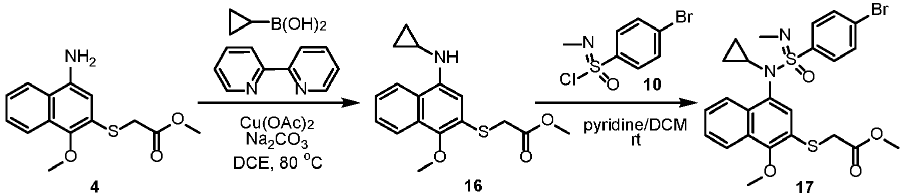

Dissolving an intermediate 4 (1.5mmol) and 2,2' -dipyridyl (1.5mmol) in dry DCE ( 7 mL), and adding cyclopropylboronic acid (3.0mmol), copper acetate (1.5mmol) and sodium carbonate (3.3mmol) in sequence, heating to 80 degrees and stirring for 24 hours. Detecting the forming of a product 16 by LC-MS. Purifying the intermediate 16 (153 mg) by silica gel column chromatography. Dissolving the intermediate 16 (0.48 mmol) in DCM (0.4 mL) and pyridine (0.2 mL) and stirring in an ice-water bath, adding an intermediate 10 (0.50 mmol), then slowly returning to room temperature. Detecting the forming of an intermediate 17by LC-MS. Purifying the intermediate 17 (170 mg) by silica gel column chromatography.

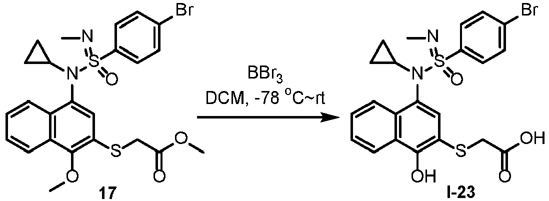

Using intermediate 17 (0.31 mmol) as raw material, preparing the compound **I-23** (37 mg) by performing demethylation with boron tribromide according to the synthesis method of the compound **I-2 .**

Taking the purified product **I-23** for a structural characterization, LC-MS (ESI) m/z: 519, 521 [MH] ⁻.

### Embodiment 24 : Synthesis and characterization of compound I-24

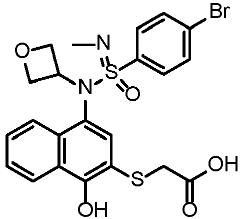

According to the synthesis method of compound **I-23** , preparing the compound **I-24** by replacing 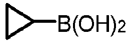 therein with

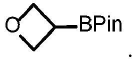

Taking the purified product **I-23** for a structural characterization, LC-MS (ESI) m/z: 535, 537 [MH]⁻.

### Embodiment 25 : Synthesis and characterization of compound I-25

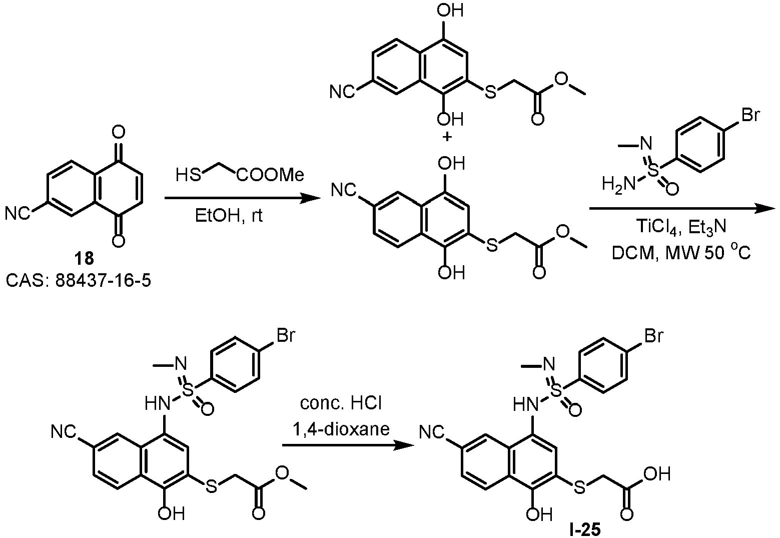

Using the known compound **18** (CAS: 88437-16-5) (5.0mmol) as raw material, preparing the compound **I-25** according to the synthesis method in the literature (J. Med. Chem. 2012, 55, 1978-1998) .

Taking the purified product **I-25** for a structural characterization: LC-MS (ESI) m/z: 504, 506 [MH] ⁻.

### Embodiment 26 : Synthesis and characterization of compound I-26

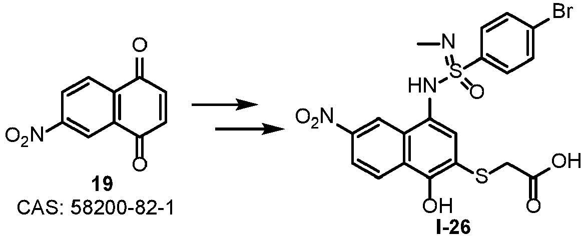

Using the known compound **19** (CAS: 58200-82-1) (5.0mmol) as raw material, preparing the compound **I-26** according to the synthesis method in the literature (J. Med. Chem. 2012, 55, 1978-1998) .

Taking the purified product **I-26** for a structural characterization: LC-MS (ESI) m/z: 524, 526 [MH] ⁻.

### Test Example 1. Functional test of mitophagy inducer

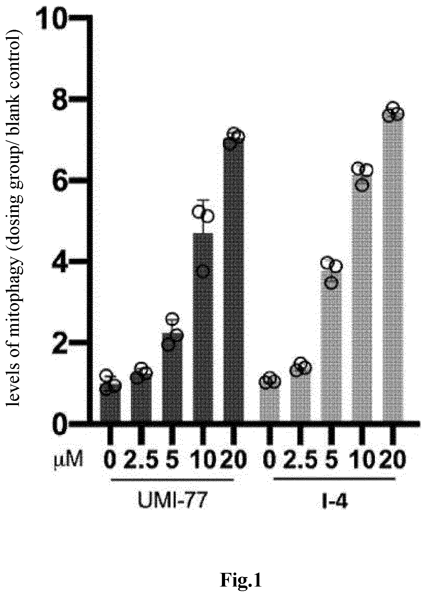

Seeding human embryonic kidney transformed cells HEK293T-mtkeima cells in a 96-well black ELISA Plate at 1.5*10⁵ cells/ml, 100 µl per well; after 25 hours, adding **UMI-77** and compounds **1-1-1-26** respectively, and setting up 3 replicates; under 37°C, 5% CO₂ incubation conditions, taking picture with biotekcytation5 every few hours for a total of 20 hours. Taking the bright field as the focusing channel, and taking 9 pictures per well, and processing the pictures by using the instrument software and converting into the degree of mitophagy strength, exemplifying by UMI-77 and Compound I-4, the results are shown in Fig. 1.

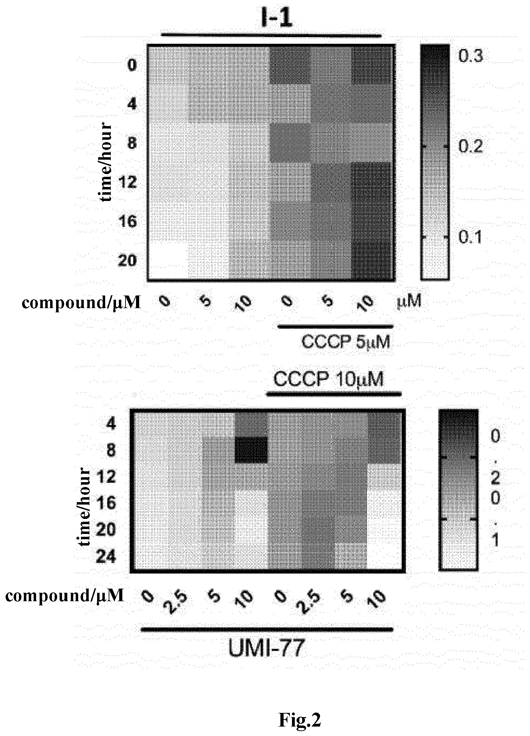

Seeding human embryonic kidney transformed cells HEK293T-mtkeima cells in a 96-well black ELISA Plate at 1.5*10 ⁵ cells/ml, 100 µl per well; after 24 hours, adding 5uM or 10uM CCCP (Carbonyl cyanide 3-chlorophenylhydrazone), after 1 hour, adding **UMI-77** and compounds **I-1-1-26** respectively, and setting up 3 replicates; under 37°C, 5% CO₂ incubation conditions, taking picture with biotekcytation5 every few hours for a total of 20 hours. Taking the bright field as the focusing channel, and taking 9 pictures per well, and processing the pictures by using the instrument software, exemplifying by UMI-77 and compounds I-1, I-4, and I-5, and the results are shown in Figs. 2, 3, and 4 (the colour depth of the blocks represents the strength of mitophagy).

As shown in Fig. 1, on cells not treated with CCCP, compound I-4 induces mitophagy more strongly than UMI-77.

As shown in Fig. 2 , the human embryonic kidney transformed cell HEK293T-mtkeima system is treated with 5uM or 10uM CCCP to induce mitochondrial damage, then adding compound **I-1** compared with no CCCP treatment and only adding the test compound, the color of the block is darker, indicating that the compound **I-1** is more likely to induce autophagy in damaged mitochondria. As for **UMI-77** , under the same conditions, the color blocks of damaged mitochondria and undamaged mitochondria are both darker, indicating that **UMI-77** is unable to selectively induce autophagy in damaged mitochondria.

The ability of other compounds to induce mitophagy is shown in Table 1:

**Table 1**

| Compound number | Ability to induce mitophagy | Compound number | Ability to induce mitophagy |
|---|---|---|---|
| **I-1** | +++ | **I-19** | +++ |
| **I-2** | ++ | **I-20** | ++ |
| **I-3** | ++ | **I-21** | +++ |
| **I-4** | +++ | **I-22** | ++ |
| **I-5** | +++ | **UMI-77** | + |
| **I-6** | +++ | | |
| **I-8** | ++ | | |
| **I-9** | ++ | | |
| **I-10** | ++ | | |
| **I-11** | ++ | | |
| **I-12** | +++ | | |
| **I-13** | ++ | | |
| **I-14** | ++ | | |
| **I-17** | +++ | | |
| **I-18** | ++ | | |

| | | | |
|---|---|---|---|
| "+++" indicates that the compound has a strong ability to induce mitophagy (number of cells in which mitophagy occurs / number of total cells > 0.30 ), and "++" indicates that the compound has a moderate ability to induce mitophagy ( number of cells in which mitophagy occurs / number of total cells is in the range of 0.2 ~ 0.3 ), "+" indicates that the compound has a weak ability to induce mitophagy (number of cells in which mitophagy occurs / number of total cells is in the range of 0.1 ~ 0.2 ), "NA" indicates that the compound is not detected with the ability to induce mitophagy . Note: The HEK293T-mtkeima cells used in the experiments are HEK293T-mtkeima cells obtained according to the method mentioned in the literature [Cen, X. et al. Nat Commun 11, 5731 (2020). ], by using lentiviral packaging technology, and stably expressing mtkeima protein in HEK293T cells. The CCCP used in the experiments is purchased from TargetMol (Item No. T7081). | | | |

### Test example 2, in vitro liver microsome stability test

Selecting Ketanserin as the reference compound. The specific method is as follows:
Preparing 0.1M K₃PO₄ (pH 7.4) buffer and 3 ×NADPH stock solution (6mM, 5mg/mL), and preheating in a 37°C water bath; preparing of test compound and control compound spiking solution: adding 5µL compound stock solution (10 nM) into 95 µL acetonitrile; preparing of 1.5 µM spiking solution in microsomes (0.75 mg/mL): adding 1.5 µL spiking solution and 18.75 µL liver microsomes solution (20 mg/mL) into 479.75 µL K₃PO₄ buffer; taking 30 µL spiking solution in microsomes to the multi-well plate and incubating at 37°C for 5 minutes; adding 15 µL NADPH stock solution into each well to start the reaction, and timing; respectively at 0min, 5min, 15min, 30min and 45min, adding 150 µL of IS-containing acetonitrile solution to terminate the reaction; shaking for 10 mins and then centrifuging at 6000 rpm for 15 mins; taking 80 µL of the supernatant from each well for LC/MS detection and calculating T_{1/2} . Some test results are shown in Figure 5.

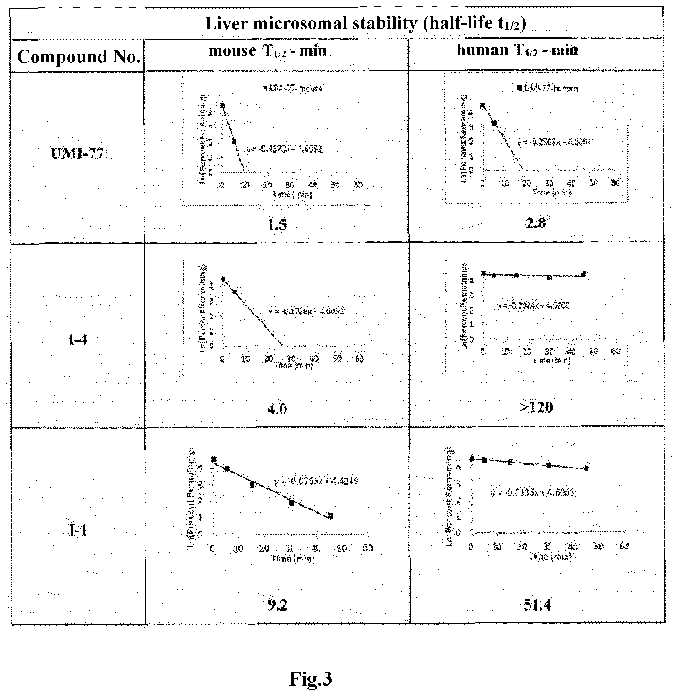

As shown in Fig. 3, the in vitro liver microsome stability experiment of **UMI-77** shows that **UMI-77** is unstable in the environment of mice or human liver microsomes and will be eliminated rapidly; compounds **I-1** and **I-4** show relatively superior stability.

Note: The mice and human liver microsomes used in the experiments are purchased from Xenotech .

Those of ordinary skill in the art can understand that the above-mentioned embodiments are specific examples for implementing the present invention, and in practical applications, various changes can be made in form and details without departing from the spirit and scope of the present invention.

## Claims

1. A compound having a structure represented by general formula (I), or a pharmaceutically acceptable salt, stereoisomer, solvate or prodrug thereof, 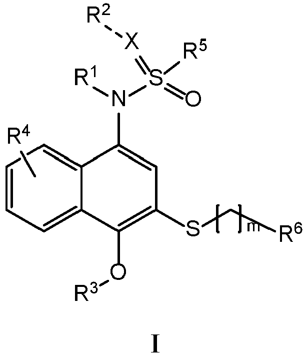
wherein X is oxygen or nitrogen;
R³ is hydrogen, 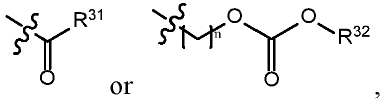 wherein R³¹ and R³² are each independently C_{1~6} alkyl or C_{1~6} cycloalkyl, n is 1~4 ;
the number of R⁴ is 0~5, and when the number of R ⁴ is not 0, each is independently selected from halogen, nitro, nitrile , -N⁺(R⁴⁻¹)₃, C_{1~6} haloalkyl, -C(O)OR⁴⁻¹ , -C(O)R⁴⁻¹ , -C(O)N(R⁴⁻¹R^{4-1a}) , -S(O)₂R⁴⁻¹ , - S(O)R⁴⁻¹, -N=C(R⁴⁻¹R^{4-1a}) , wherein R⁴⁻¹ and R^{4-1a} are each independently hydrogen, C_{1~6} alkyl, C₂₋₆ alkenyl, C₂₋₆ alkynyl , C₁₋₆ alkyl with at least one hydrogen substituted by halogen , C₂₋₆ alkenyl with at least one hydrogen substituted by halogen, or C₂₋₆ alkynyl with at least one hydrogen substituted by halogen;
R⁵ is phenyl, naphthyl, 5- or 6-membered monocyclic heteroaryl, 8- to 10-membered fused bicyclic heteroaryl, phenyl with at least one hydrogen atom is substituted by R ⁵⁻¹ , naphthyl with at least one hydrogen atom is substituted by R ⁵⁻¹, 5- or 6-membered monocyclic heteroaryl with at least one hydrogen atom substituted by R⁵⁻¹ , 8- to 10-membered fused bicyclic heteroaryl with at least one hydrogen atom substituted by R⁵⁻¹ , wherein R⁵⁻¹ is hydrogen, halogen, nitro, nitrile , hydroxyl, C_{1~6} alkyl, C₃-₆ cycloalkyl, C_{1~6} alkoxy , -N(R^{5-1a}R5^{-1b}), phenyl, C_{1~6} haloalkyl, C_{1~6} haloalkoxy, - C(O)OR⁵⁻¹¹, -C(O)R⁵⁻¹¹ , -C(O)N(R^{5-1a}R^{5-1b}), -S(O)₂R⁵⁻¹¹ , -S(O)R⁵⁻¹¹ , - OC(O)R⁵⁻¹¹ , -OC(O)OR⁵⁻¹¹ or 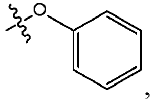 wherein R⁵⁻¹¹ , R^{5-1a} and R^{5-1b} are each independently hydrogen, C_{1~6} alkyl, three- to six-membered cycloalkyl, C_{2 -6} alkenyl, C₂₋₆ alkynyl, C_{1~6} alkyl with at least one hydrogen substituted by halogen, C₂₋₆ alkenyl with at least one hydrogen substituted by halogen, C₃-₆ cycloalkyl with at least one hydrogen substituted by halogen , C₂₋₆ alkynyl with at least one hydrogen substituted by halogen;
R⁶ is -C(O)OR⁶⁻¹ , 5 or 6-membered monocyclic heteroaryl , wherein R⁶⁻¹ is hydrogen, C_{1~6} alkyl, three to six-membered cycloalkyl, C₂₋₆ alkenyl, C₂₋₆ alkynyl , C_{1~6} alkyl with at least one hydrogen substituted by halogen , C₂₋₆ alkenyl with at least one hydrogen substituted by halogen, C_{3~6} cycloalkyl with at least one hydrogen substituted by halogen, C₂₋₆ alkynyl with at least one hydrogen substituted by halogen;
m is 1~6 ; and
when X is oxygen, a dotted line does not exist and R² does not exist, R¹ is C_{1~6} alkyl, three- to six-membered cycloalkyl, three- to six-membered epoxyalkyl, phenyl, C_{1~6} alkyl with at least one hydrogen substituted by R¹⁻¹ , and phenyl with at least one hydrogen substituted by R¹⁻¹ , wherein R¹⁻¹ is hydroxyl, halogen, amino or C_{1~6} alkoxy;
when X is nitrogen, the dotted line is a single bond,
R¹ is hydrogen, C_{1 ~ 6} alkyl, three- to six-membered cycloalkyl, three-to six-membered epoxyalkyl, phenyl, C_{1~6} alkyl with at least one hydrogen substituted by R¹⁻¹ , phenyl with at least one hydrogen substituted by R¹⁻², wherein R¹⁻² is hydroxyl, halogen, amino or C_{1~6} alkoxy,
R² is a C_{1~6} alkyl, a three- to six-membered cycloalkyl, a three- to six-membered epoxyalkyl, or a C_{1~6} alkyl with at least one hydrogen substituted by R²⁻¹ , wherein R²⁻¹ is hydroxyl, halogen, amino or C_{1~6} alkoxy;
when X is nitrogen and R¹ and R² are independently C₁₋₆ alkyl, R¹ and R² can be bonded to form a 4- to 8-membered ring.

2. The compound or the pharmaceutically acceptable salt, stereoisomer, solvate or prodrug thereof according to claim 1, wherein
X is oxygen or nitrogen;
R³ is hydrogen, 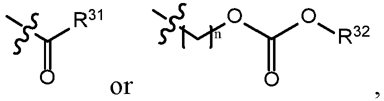 wherein R³¹ and R³² are each independently C_{1~4} alkyl or C_{1~4} cycloalkyl, n is 1 or 2;
when the number of R⁴ is not 0, each is independently selected from halogen, nitro, nitrile , -N ⁺(R⁴⁻¹)₃ , C _{1~4} haloalkyl, -C(O)OR⁴⁻¹ , -C(O)R⁴⁻¹ , -C(O)N(R⁴⁻¹R^{4-1a}) , -S(O)₂R⁴⁻¹ , -S(O)R⁴⁻¹ , -N=C (R⁴⁻¹R^{4-1a} ), wherein R⁴⁻¹ and R^{4-1a} are each independently hydrogen, C₁₋₄ alkyl, C₂₋₄ alkenyl, C₂₋₄ alkynyl ;
R⁵ is phenyl, naphthyl, 5- or 6-membered monocyclic heteroaryl, phenyl with at least one hydrogen atom substituted by R⁵⁻¹ , naphthyl with at least one hydrogen atom substituted with R⁵⁻¹ , 5- or 6-membered monocyclic heteroaryl with at least one hydrogen atom substituted by R^{5-1 ,} wherein R⁵⁻¹ is hydrogen, halogen, nitro, nitrile , hydroxyl, C_{1~4} alkyl, or three- to six-membered cycloalkyl, C_{1~4} alkoxy, -N(R^{5-1a} R^{5-1b}), phenyl, C_{1~4}haloalkyl, C_{1~4} haloalkoxy, -C(O)OR⁵⁻¹¹ , -C(O)R⁵⁻¹¹ , -C(O)N(R^{5-1a}R^{5-1b} ), -S(O)₂R⁵⁻¹¹ , -S(O)R⁵⁻¹¹ , - OC(O)R⁵⁻¹¹ , -OC(O)OR⁵⁻¹¹ or 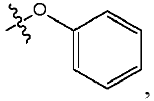 wherein R⁵⁻¹¹ , R^{5-1a} and R^{5-1b} are each independently hydrogen, C_{1~4} alkyl, three- to six -membered cycloalkyl, C₂₋₄ alkenyl , C₂₋₄ alkynyl , C_{1~4} alkyl with at least one hydrogen substituted by halogen;
R⁶ is -C(O)OR⁶⁻¹ , 5 or 6-membered monocyclic heteroaryl , wherein R ⁶⁻¹ is hydrogen, C _{1~4} alkyl, three to six-membered cycloalkyl, C _{1~4} alkyl with at least one hydrogen substituted by halogen;
m is 1~4 ; and
when X is oxygen, the dotted line does not exist, R² does not exist, and R¹ is C_{1~4} alkyl, three- to six-membered cycloalkyl, three- to six-membered epoxyalkyl, phenyl, C_{1~4} alkyl with at least one hydrogen substituted by R¹⁻¹, and phenyl with at least one hydrogen substituted by R¹⁻¹, wherein R¹⁻¹ is hydroxyl, halogen, amino, or C_{1~4} alkoxy;
when X is nitrogen, the dotted line is a single bond,
R¹ is hydrogen, C_{1~4} alkyl, three- to six-membered cycloalkyl, three-to six-membered epoxyalkyl, phenyl, C_{1~4} alkyl with at least one hydrogen substituted by R¹⁻¹ , phenyl with at least one hydrogen substituted by R¹⁻² , wherein R¹⁻² is hydroxyl, halogen, amino or C_{1~4} alkoxy ,
R² is C_{1~4} alkyl, three- to six-membered cycloalkyl, three- to six-membered epoxyalkyl, or C_{1~4} alkyl with at least one hydrogen substituted by R²⁻¹ , wherein R²⁻¹ is hydroxyl, halogen, amino or C_{1~4} alkoxy ;
when X is nitrogen and R¹ and R² are independently C_{1~4} alkyl, R¹ and R² can be bonded to form a 4 ~ 8-membered ring.

3. The compound or the pharmaceutically acceptable salt, stereoisomer , solvate or prodrug thereof according to claim 1, wherein
X is oxygen or nitrogen;
R³ is hydrogen, 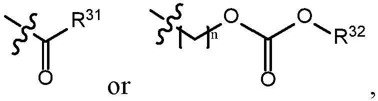 wherein R³¹ and R³² are independently methyl, ethyl, n-propyl or isopropyl, n is 1;
when the number of R⁴ is not 0 , each is independently selected from fluorine, chlorine, bromine, iodine, nitro, nitrile, fluoromethyl , fluoroethyl , fluoro-n-propyl , fluoroisopropy , chloromethyl, chloroethyl, chloro-n-propyl, chloroisopropyl, bromomethyl, bromoethyl, bromo-n-propyl, bromoisopropyl, iodomethyl, iodoethyl, iodo-n-propyl or iodoisopropyl;
R⁵ is phenyl, naphthyl, 5-membered monocyclic heteroaryl, and phenyl with at least one hydrogen atom is substituted by R⁵⁻¹ , wherein R⁵⁻¹ is fluorine, chlorine, bromine, iodine , methoxy, ethoxy, n-propoxy , isopropoxy, n-butoxy , tert-butoxy , sec-butoxy or isobutoxy , phenyl, fluoromethyl , fluoroethyl, fluoro-n-propyl , fluoroisopropyl , chloromethyl, chloroethyl, chloro-n-propyl, chloroisopropyl , bromomethyl, bromoethyl, bromo-n-propyl , bromoisopropyl , iodomethyl, iodoethyl, iodo-n-propyl , iodoisopropyl , fluoromethoxy, fluoroethoxy , fluoro-n-propoxy , fluoroisopropoxy, chloromethoxy, chloroethoxy, chloro-n-propoxy, chloroisopropoxy, bromomethoxy, bromoethoxy, bromo-n-propoxy, bromoisopropoxy, iodooxymethyl, iodoethoxy, iodo-n-propoxy, iodoisopropoxy or 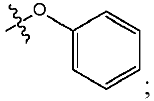
R⁶ is -C(O)OR⁶⁻¹ , five-membered nitrogen-containing monocyclic heteroaryl, wherein R⁶⁻¹ is hydrogen, methyl, ethyl, n-propyl, isopropyl, 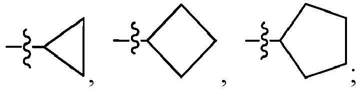
m is 1; and
when X is oxygen, the dotted line does not exist, R² does not exist, and R¹ is C_{1~4} alkyl, three- to six-membered cycloalkyl, three- to six-membered epoxyalkyl, phenyl, C_{1~4} alkyl with at least one hydrogen substituted by R¹⁻¹ , phenyl with at least one hydrogen substituted by R¹⁻¹ , wherein R¹⁻¹ is hydroxyl, halogen, amino or C_{1~4} alkoxy ,
when X is oxygen, the dotted line does not exist, R² does not exist, and R¹ is methyl, ethyl, n-propyl, or isopropyl, 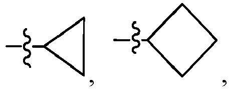 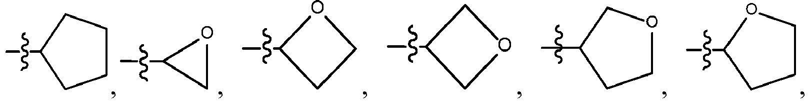 phenyl, methyl with at least one hydrogen substituted by hydroxyl, ethyl with at least one hydrogen substituted by hydroxyl, n-propyl with at least one hydrogen substituted by hydroxyl, isopropyl with at least one hydrogen substituted by hydroxyl;
when X is nitrogen, the dotted line is a single bond,
R¹ is hydrogen, methyl, ethyl, n-propyl , isopropyl, 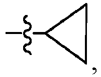 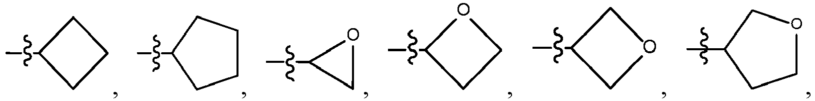 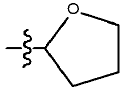 or phenyl,
R² is methyl, ethyl, n-propyl , isopropyl, 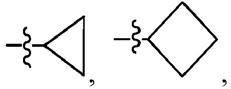 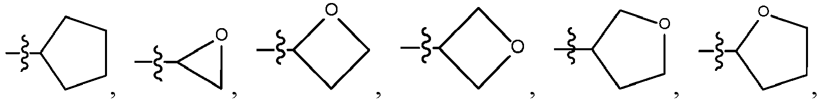 methyl with at least one hydrogen substituted by hydroxyl, ethyl with at least one hydrogen substituted by hydroxyl, n-propyl with at least one hydrogen substituted by hydroxyl or isopropyl with at least one hydrogen substituted by hydroxyl;
when X is nitrogen and R¹ and R² are independently any one of methyl, ethyl, n-propyl or isopropyl, R¹ and R² can be bonded to form a 4-to 8-membered ring.

4. The compound or the pharmaceutically acceptable salt, stereoisomer, solvate or prodrug thereof according to any one of claims 1 to 3, wherein
R³ is hydrogen, 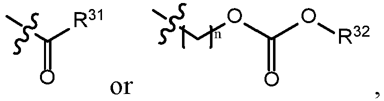 wherein R³¹ and R³² are each independently methyl, n-propyl or isopropyl, and n is 1;
and/or, when the number of R ⁴ is not 0, each is independently selected from fluorine, chlorine, bromine, iodine , nitro, and nitrile ;
and/or, R⁵ is phenyl, naphthyl, thienyl or phenyl with at least one hydrogen atom substituted by R⁵⁻¹ , wherein R⁵⁻¹ is fluorine, chlorine, bromine, iodine, phenyl, fluoromethyl or 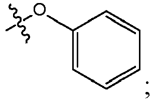
and/or, R⁶ is -C(O)OH , triazolyl or tetrazolyl .

5. The compound or the pharmaceutically acceptable salt, stereoisomer, solvate or prodrug thereof according to any one of claims 1 to 3, wherein X is nitrogen.

6. The compound or the pharmaceutically acceptable salt, stereoisomer, solvate or prodrug thereof according to claim 5, wherein
R¹ is hydrogen, methyl, ethyl, cyclopropanyl or cyclobutanyl;
and/or, R² is hydrogen, methyl, ethyl, phenyl, cyclopropyl, cyclobutanyl or 2-hydroxyethyl;
and/or, when R¹ is methyl or ethyl and R¹ is methyl or ethyl, R¹ and R² can be bonded to form a 5- to 6 -membered ring.

7. The compound or the pharmaceutically acceptable salt, stereoisomer, solvate or prodrug thereof according to claim 6, wherein
R⁴ is bromine, nitro or nitrile;
and/or, R⁵ is phenyl or phenyl with at least one hydrogen atom substituted by bromine.

8. The compound or the pharmaceutically acceptable salt, stereoisomer, solvate or prodrug thereof according to any one of claims 1 to 3, wherein the compound having the structure represented by the general formula (I) is selected from any one of the following compounds:
| | |
|---|---|
| 1 | 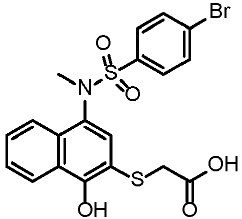 |
| 2 | 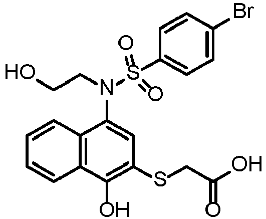 |
| 3 | 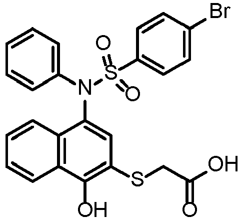 |
| 4 | 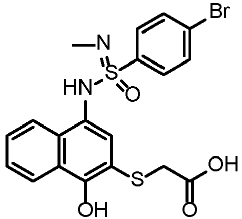 |
| 5 | 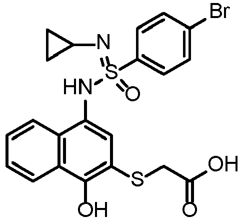 |
| 6 | 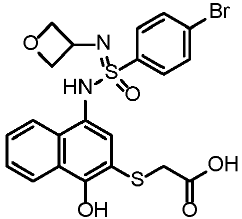 |
| 7 | 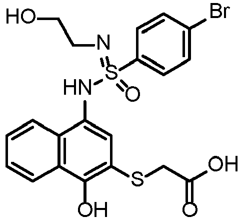 |
| 8 | 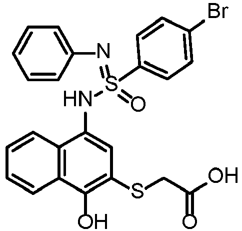 |
| 9 | 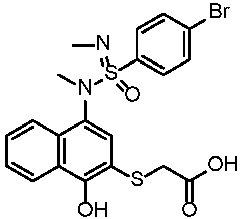 |
| 10 | 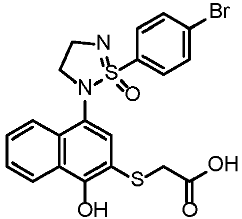 |
| 11 | 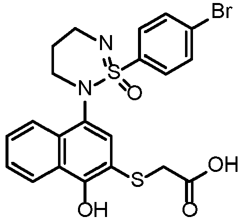 |
| 12 | 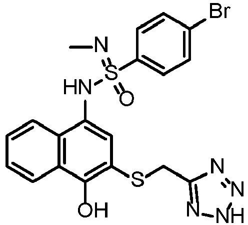 |
| 13 | 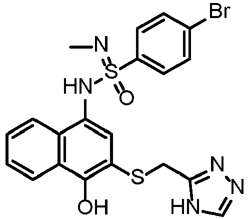 |
| 14 | 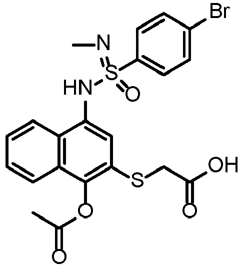 |
| 15 | 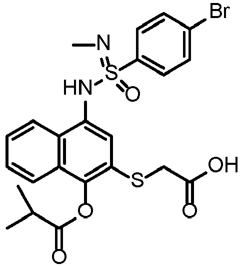 |
| 16 | 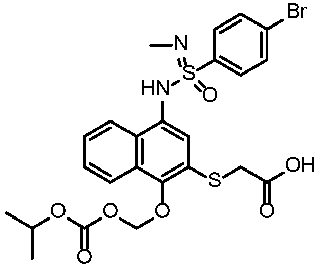 |
| 17 | 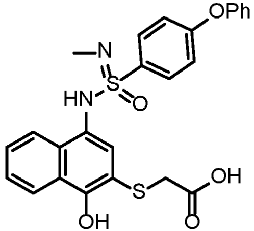 |
| 18 | 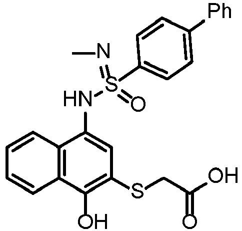 |
| 19 | 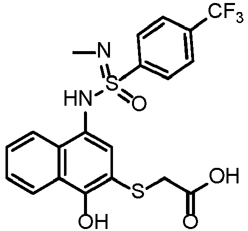 |
| 20 | 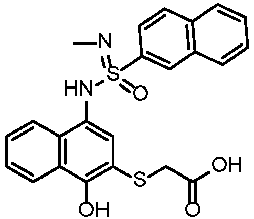 |
| 21 | 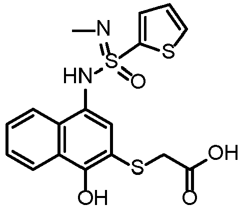 |
| 22 | 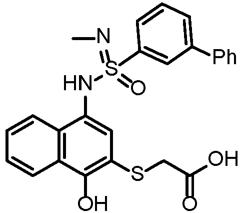 |
| 23 | 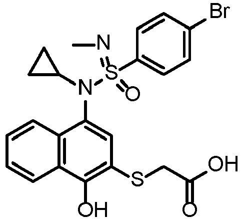 |
| 24 | 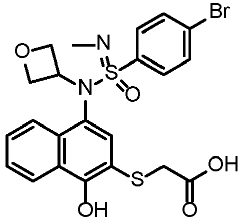 |
| 25 | 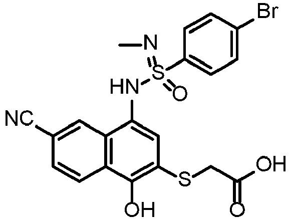 |
| 26 | 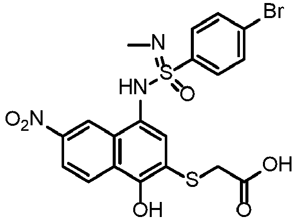 |

9. A pharmaceutical composition, comprising the compound or the pharmaceutically acceptable salt, stereoisomer, solvate or prodrug thereof according to any one of claims 1 to 8.

10. Use of the compound, or the pharmaceutically acceptable salt, stereoisomer, solvate or prodrug thereof according to any one of claims 1 to 8, or the pharmaceutical composition of claim 9 for the preparation of a mitophagy inducer .
